(19) **Europäisches Patentamt European Patent Office Office européen des brevets**

(11) **EP 4 142 675 B1**

(12) **EUROPEAN PATENT SPECIFICATION**

(45) Date of publication and mention of the grant of the patent:
**25.03.2026 Bulletin 2026/13**

(21) Application number: **21719649.2**

(22) Date of filing: **21.04.2021**

(51) International Patent Classification (IPC):
*A61K 8/02* (2006.01)   *A61K 8/19* (2006.01)
*A61K 8/73* (2006.01)   *A61K 8/81* (2006.01)
*A61K 47/02* (2006.01)   *A61K 47/32* (2006.01)
*A61K 47/38* (2006.01)   *A61Q 19/00* (2006.01)
*A61K 9/16* (2006.01)   *A61K 9/20* (2006.01)

(52) Cooperative Patent Classification (CPC):
**A61K 8/0241; A61K 8/19; A61K 8/731;
A61K 8/817; A61K 9/1611; A61K 9/1635;
A61K 9/1652; A61K 9/2009; A61K 9/2027;
A61K 9/2054; A61K 47/02; A61K 47/32;
A61K 47/38; A61Q 19/00;** A61K 2800/60

(86) International application number:
**PCT/EP2021/060361**

(87) International publication number:
**WO 2021/219458 (04.11.2021 Gazette 2021/44)**

(54) **GRANULES COMPRISING SURFACE-REACTED CALCIUM CARBONATE AS EXCIPIENT**

GRANULATE MIT OBERFLÄCHENREAGIERTEM CALCIUMCARBONAT ALS HILFSSTOFF

GRANULÉS COMPRENANT DU CARBONATE DE CALCIUM TRAITÉ PAR RÉACTION EN SURFACE COMME EXCIPIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2020 EP 20171924**

(43) Date of publication of application:
**08.03.2023 Bulletin 2023/10**

(73) Proprietor: **Omya International AG
4665 Oftringen (CH)**

(72) Inventors:
• **DE MIGUEL, Laura
4800 Zofingen (CH)**
• **CAMARGO, Javier
68220 Hésingue (FR)**
• **DIAZ QUIJANO, Carolina
4665 Oftringen (CH)**

(74) Representative: **Maiwald GmbH
Elisenhof
Elisenstraße 3
80335 München (DE)**

(56) References cited:
WO-A1-2015/181306   WO-A1-2016/150773
WO-A1-2017/093437   WO-A1-2017/220406
WO-A1-2017/220498   WO-A1-2020/058252
US-A1- 2011 151 014

**EP 4 142 675 B1**

**Description**

The present invention refers to the use of granules comprising surface-reacted calcium carbonate and one or more binder(s) as excipient in a pharmaceutical, nutraceutical, agricultural, veterinary, cosmetic, home, food, packaging or personal care product, wherein the granules have i) a weight particle size $d_{90}$ of 150 to 700 $\mu$m, as measured according to mechanical sieving, ii) a weight median particle size $d_{50}$ of 45 to 300 $\mu$m, as measured according to mechanical sieving, iii) a weight particle size $d_{10}$ of 18 to 100 $\mu$m, as measured according to mechanical sieving, and iv) a specific surface area of $\geq$ 15.0 $m^2$/g as measured by the BET nitrogen method, wherein the one or more binder(s) is/are synthetic polymers selected from methylcellulose, ethylcellulose, sodium carboxymethylcellulose, sodium crosscarmellose, hydroxypropyl methylcellulose (HPMC), hydroxypropylcellulose (HPC), ethylhydroxyethylcellulose (EHEC), polyvinyl pyrrolidone (PVP), polyethylene glycol (PEG), polyvinyl alcohols, polymethacrylates; and natural binders selected from plant gums, selected from acacia, tragacanth, sandarac, ghatti, karaya, locust bean, carnauba wax, and guar; proteins selected from gelatin, casein, collagen, animal exudates selected from shellac; beeswax, alginic acid, and mixtures thereof, and wherein the granules comprise the one or more binder(s) in an amount of from 0.25 to 35 wt.-%, based on the total dry weight of the granules.

Surface-reacted calcium carbonate powder can be used as a carrier in a great variety of applications due to its high porosity and capacity of loading/mixing active/inactive agents. Thus, surface-reacted calcium carbonate is gaining more and more importance as excipient in the production of dosage forms for pharmaceutical, nutraceutical, agricultural, veterinary, cosmetic, home, food, packaging or personal care products. In particular, methods have been developed for producing dosage forms which are in the form of a compacted material comprising surface-reacted calcium carbonate.

For examples, WO2016150773 A1 refers to a method for producing a dispersible dosage form, comprising the steps of: a) providing a functionalized calcium carbonate-comprising material, which is a reaction product of natural ground or precipitated calcium carbonate with carbon dioxide and one or more acids in an aqueous medium, wherein the carbon dioxide is formed in situ by the acid treatment and/or is supplied from an external source, b) providing at least one disintegrant; c) optionally providing at least one further formulating aid; d) mixing the functionalised calcium carbonate-comprising material of step a), the at least one disintegrant of step b) and the optionally at least one further formulating aid of step c); and e) compacting the mixture obtained in step d) by means of a roller compactor at a compaction pressure in the range from 2 to 20 bar into a ribbon; and f) milling the ribbon of step e) into granules, g) sieving of the granules of step f) by at least one mesh size.

WO2016096997 A1 refers to a method for producing a pharmaceutical delivery system, comprising the steps of: a) providing surface-reacted calcium carbonate, which is a reaction product of natural ground or precipitated calcium carbonate with carbon dioxide and one or more acids in an aqueous medium, wherein the carbon dioxide is formed in situ by the acid treatment and/or is supplied from an external source; b) providing at least one pharmaceutically active agent or pharmaceutically inactive precursor thereof; c) providing at least one formulating aid; d) mixing the surface-reacted calcium carbonate of step a), the at least one pharmaceutically active agent or pharmaceutically inactive precursor thereof of step b) and the at least one formulating aid of step c); and e) compacting the mixture obtained in step d) by means of a roller compacter at a compaction pressure in the range from 4 to 20 bar; and f) compacting the roller compacted mixture obtained in step e) for obtaining the pharmaceutical delivery system. WO2020058252 A1 refers to a particulate pharmaceutical or nutraceutical excipient comprising a) microcrystalline cellulose, and b) surface-reacted calcium carbonate, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment and/or is supplied from an external source, wherein the weight ratio of the microcrystalline cellulose to the surface-reacted calcium carbonate is from 99.9:0.1 to 50:50. WO2017093437 A1 refers to a method for the production of granules comprising surface-reacted calcium carbonate, characterized by the steps of a) providing surface-reacted calcium carbonate, wherein the surface-reacted calcium carbonate is a reaction product of natural ground or precipitated calcium carbonate with carbon dioxide and one or more $H_3O^+$ ion donors in an aqueous medium, wherein the carbon dioxide is formed in-situ by the $H_3O^+$ ion donor treatment and/or is supplied from an external source; b) providing one or more active ingredient(s) in liquid form, c) saturating the surface-reacted calcium carbonate with the one or more active ingredient(s) in liquid form, d) providing one or more binder, and e) combining the saturated surface-reacted calcium carbonate obtained in step c) with the one or more binder of step d) under agitation in an agitation device. WO2015181306 A1 refers to a method for the production of granules comprising surface-reacted calcium carbonate, characterized by the steps of a) providing surface-reacted calcium carbonate, wherein the surface-reacted calcium carbonate is a reaction product of natural ground or precipitated calcium carbonate with carbon dioxide and one or more acids, wherein the carbon dioxide is formed in situ by the acid treatment and/or is supplied from an external source; b) saturating the surface-reacted calcium carbonate with one or more liquids; c) providing one or more binder; d) combining the liquid saturated surface-reacted calcium carbonate with the one or more binder under agitation in an agitation device; e) removing the liquid from the mixture of step d).

However, the most popular method for preparing solid dosage forms is direct compression as it provides the most effective and least complex way to produce such dosage forms. Due to the bad flowability characteristics of the surface-reacted calcium carbonate powder, such powder is however not suitable for direct compression and loading/mixing.

Thus, there is a continuous need for materials that can be used as excipient in a pharmaceutical, nutraceutical, agricultural, veterinary, cosmetic, home, food, packaging or personal care product. Furthermore, it is desired to provide an excipient comprising surface-reacted calcium carbonate due to its advantageous loading and release characteristics.

It is thus an object of the present invention to provide a material that can be used as excipient in a pharmaceutical, nutraceutical, agricultural, veterinary, cosmetic, home, food, packaging or personal care product. Another object may also be seen in the provision of an excipient comprising surface-reacted calcium carbonate due to its advantageous loading and release characteristics.

One or more of the foregoing and other problems are solved by the subject-matter as defined herein in the independent claim. Advantageous embodiments of the present invention are defined in the corresponding sub-claims.

The present invention thus relates to the use of granules comprising surface-reacted calcium carbonate and one or more binder(s) as excipient in a pharmaceutical, nutraceutical, agricultural, veterinary, cosmetic, home, food, packaging or personal care product, wherein the granules have i) a weight particle size $d_{90}$ of 150 to 700 $\mu$m, as measured according to mechanical sieving, ii) a weight median particle size $d_{50}$ of 45 to 300 $\mu$m, as measured according to mechanical sieving, iii) a weight particle size $d_{10}$ of 18 to 100 $\mu$m, as measured according to mechanical sieving, and iv) a specific surface area of $\geq$ 15.0 m$^2$/g as measured by the BET nitrogen method, wherein the one or more binder(s) is/are synthetic polymers selected from methylcellulose, ethylcellulose, sodium carboxymethylcellulose, sodium crosscarmellose, hydroxypropyl methylcellulose (HPMC), hydroxypropylcellulose (HPC), ethylhydroxyethylcellulose (EHEC), polyvinyl pyrrolidone (PVP), polyethylene glycol (PEG), polyvinyl alcohols, polymethacrylates; and natural binders selected from plant gums, selected from acacia, tragacanth, sandarac, ghatti, karaya, locust bean, carnauba wax, and guar; proteins selected from gelatin, casein, collagen, animal exudates selected from shellac; beeswax, alginic acid, and mixtures thereof, and wherein the granules comprise the one or more binder(s) in an amount of from 0.25 to 35 wt.-%, based on the total dry weight of the granules.

According to one embodiment, the surface-reacted calcium carbonate is a reaction product of natural ground or precipitated calcium carbonate with carbon dioxide and one or more $H_3O^+$ ion donors in an aqueous medium, wherein the carbon dioxide is formed in-situ by the $H_3O^+$ ion donor treatment and/or is supplied from an external source.

According to another embodiment, the natural ground calcium carbonate is selected from calcium carbonate containing minerals selected from the group comprising marble, chalk, limestone and mixtures thereof; and that the precipitated calcium carbonate is selected from the group comprising precipitated calcium carbonates having aragonitic, vateritic or calcitic mineralogical crystal forms or mixtures thereof.

According to yet another embodiment, the surface-reacted calcium carbonate has i) a BET specific surface area of from 20 m$^2$/g to 450 m$^2$/g, preferably from 20 m$^2$/g to 250 m$^2$/g, more preferably from 30 m$^2$/g to 160 m$^2$/g, most preferably from 40 m$^2$/g to 150 m$^2$/g, still more preferably from 40 m$^2$/g to 140 m$^2$/g measured using the nitrogen and BET method according to ISO 9277:2010, and/or ii) a volume median particle diameter $d_{50}$ of from 1 $\mu$m to 50 $\mu$m, preferably from 1 to 45 $\mu$m, more preferably from 2 to 30 $\mu$m, and/or iii) an intra-particle intruded specific pore volume within the range from 0.15 to 1.35 cm$^3$/g, preferably from 0.30 to 1.30 cm$^3$/g, more preferably from 0.30 to 1.25 cm$^3$/g, and most preferably from 0.30 to 0.90 cm$^3$/g calculated from a mercury intrusion porosimetry measurement.

According to another embodiment, the granules comprise the one or more binder(s) in an amount of from 0.5 to 15 wt.-%, more preferably of from 0.5 to 10 wt.-%, even more preferably of from 1.0 to 10 wt.-%, most preferably of from 1.5 to 10 wt.-%, based on the total dry weight of the granules.

According to yet another embodiment, the granules comprise and/or are mixed with at least one active ingredient and/or inactive precursor thereof, preferably selected from the group comprising fragrances, flavours, herbal extracts and oils, fruit extracts and oils, nutrients, trace minerals, repellents, food, cosmetics, flame retardants, enzymes, macromolecules, pesticides, fertilizers, preserving agents, antioxidants, reactive chemicals, pharmaceutical and/or nutraceutical and/or veterinary active agents or pharmaceutical and/or nutraceutical and/or veterinary inactive precursors of synthetic origin, semi-synthetic origin, natural origin thereof, and mixtures thereof, and/or one or more lubricant(s) and/or one or more disintegrant(s).

According to one embodiment, the granules comprise the at least one active ingredient and/or inactive precursor thereof in an amount from 0.5 to 80 wt.-%, preferably of from 10.0 to 70 wt.-% and most preferably of from 20 to 60 wt.-%, based on the total dry weight of the granules.

According to another embodiment, the granules are obtained under agitation in an agitation device, preferably an agitation device selected from Eirich mixers, fluidized bed dryers/granulators, plate granulators, table granulators, drum granulators, disc granulators, dish granulators, ploughshare mixer, vertical or horizontal mixers, high or low shear mixer, high speed blenders and rapid mixer granulators.

According to yet another embodiment, the granules have an intra-granular specific pore volume within the range from 0.15 to 2.75 cm$^3$/g, preferably from 0.30 to 2.50 cm$^3$/g, and most preferably from 0.40 to 2.00 cm$^3$/g, calculated from a mercury intrusion porosimetry measurement.

According to one embodiment, the granules are compressed in a compression process into mini-tablets or tablets.

According to another embodiment, the granules are compressed in a direct compression process, preferably using a force in the range from 1 to 40 kN, more preferably from 1.5 to 30 kN and most preferably from 1.5 to 25 kN.

According to yet another embodiment, the granules are filled into capsules or pliable packagings such as a sachet or flowpack, doypack, stickpack, and the like.

According to one embodiment, the granules provide an improved flowability and/or compactability.

According to another embodiment, the improvement is achieved if the ratio of hardness [N] to compression force [kN] (hardness/compression force) is at least 16, preferably at least 20.

It should be understood that for the purpose of the present invention the following terms have the following meaning.

The term "excipient" refers to a non active material/ingredient that is formulated alongside an active ingredient in compositions typically used in pharmaceutical, nutraceutical, agricultural, veterinary, cosmetic, home, food, packaging or personal care products. The excipients are intentionally added into the composition in order to enable active ingredients to be applied to the user in the correct form and act as the vehicle for the active ingredients. It is appreciated that the excipient has no medicinal properties.

The term "surface-reacted" in the meaning of the present application shall be used to indicate that a material has been subjected to a process comprising partial dissolution of said material upon treatment with an $H_3O^+$ ion donor (e.g., by use of water-soluble free acids and/or acidic salts) in aqueous environment followed by a crystallization process which may occur in the absence or presence of further crystallization additives.

An "$H_3O^+$ ion donor" in the context of the present invention is a Brønsted acid and/or an acid salt, i.e. a salt containing an acidic hydrogen.

The term "acid" as used herein refers to an acid in the meaning of the definition by Brønsted and Lowry (e.g., $H_2SO_4$, $HSO_4^-$).

In the meaning of the present invention "water-insoluble" materials are defined as materials which, when mixed with deionised water and filtered on a filter having a 0.2 $\mu$m pore size at 20°C to recover the liquid filtrate, provide less than or equal to 0.1 g of recovered solid material following evaporation at 95 to 100°C of 100 g of said liquid filtrate. "Water-soluble" materials are defined as materials leading to the recovery of greater than 0.1 g of recovered solid material following evaporation at 95 to 100°C of 100 g of said liquid filtrate.

"Natural ground calcium carbonate" (GCC) in the meaning of the present invention is a calcium carbonate obtained from natural sources, such as limestone, marble, or chalk, and processed through a wet and/or dry treatment such as grinding, screening and/or fractionating, for example, by a cyclone or classifier.

"Precipitated calcium carbonate" (PCC) in the meaning of the present invention is a synthesised material, obtained by precipitation following reaction of carbon dioxide and lime in an aqueous, semi-dry or humid environment or by precipitation of a calcium and carbonate ion source in water. PCC may be in the vateritic, calcitic or aragonitic crystal form.

The BET specific surface area in the meaning of the present invention is defined as the surface area of the particles divided by the mass of the particles. As used therein the specific surface area is measured by adsorption using the BET isotherm (ISO 9277:2010) and is specified in $m^2/g$.

A "dried" material is obtained by a drying process according to which at least a portion of water is removed from a material to be dried such that a constant weight of the obtained "dried" material at 200°C is reached.

The term "compression" in the meaning of the present invention means a reduction in volume and an increase of the hardness of the excipient which is obtained under pressure.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably. This e.g. means that, unless the context clearly dictates otherwise, the term "obtained" does not mean to indicate that e.g. an embodiment must be obtained by e.g. the sequence of steps following the term "obtained" though such a limited understanding is always included by the terms "obtained" or "defined" as a preferred embodiment.

According to the present invention, it has been found that granules comprising surface-reacted calcium carbonate and one or more binder(s), can be used as excipient in a pharmaceutical, nutraceutical, agricultural, veterinary, cosmetic, home, food, packaging or personal care product.

In the following, it is referred to further details of the present invention and especially the foregoing granules comprising surface-reacted calcium carbonate and one or more binder(s).

One requirement of the present invention is that the surface-reacted calcium carbonate is in form of granules having a very specific particle size distribution.

In particular, the granules have

i) a weight particle size $d_{90}$ of 150 to 700 $\mu$m, as measured according to mechanical sieving,
ii) a weight median particle size $d_{50}$ of 45 to 300 $\mu$m, as measured according to mechanical sieving, and
iii) a weight particle size $d_{10}$ of 18 to 100 $\mu$m, as measured according to mechanical sieving.

For example, the granules have

i) a weight particle size $d_{90}$ of 150 to 600 $\mu$m, as measured according to mechanical sieving,
ii) a weight median particle size $d_{50}$ of 50 to 200 $\mu$m, as measured according to mechanical sieving, and
iii) a weight particle size $d_{10}$ of 20 to 90 $\mu$m, as measured according to mechanical sieving.

Furthermore, it is required that the granules have a specific BET specific surface area. In particular, the granules have a specific surface area of $\geq$ 15.0 $m^2$/g as measured by the BET nitrogen method. For example, the granules have a specific surface area of 15.0 to 200.0 $m^2$/g as measured by the BET nitrogen method.

Additionally, the granules have an intra-granular specific pore volume within the range from 0.15 to 2.75 $cm^3$/g, preferably from 0.30 to 2.50 $cm^3$/g, and most preferably from 0.40 to 2.00 $cm^3$/g, calculated from a mercury intrusion porosimetry measurement.

The granules comprise surface-reacted calcium carbonate.

The surface-reacted calcium carbonate is a reaction product of natural ground calcium carbonate or precipitated calcium carbonate with carbon dioxide and one or more $H_3O^+$ ion donors in an aqueous medium, wherein the carbon dioxide is formed in-situ by the $H_3O^+$ ion donors treatment and/or is supplied from an external source. Preferably, the surface-reacted calcium carbonate is a reaction product of natural ground calcium carbonate or precipitated calcium carbonate with carbon dioxide and one or more $H_3O^+$ ion donors in an aqueous medium, wherein the carbon dioxide is formed in-situ by the $H_3O^+$ ion donors treatment.

A $H_3O^+$ ion donor in the context of the present invention is a Brønsted acid and/or an acid salt.

In a preferred embodiment of the invention ,the surface-reacted calcium carbonate is obtained by a process comprising the steps of: (a) providing a suspension of natural or precipitated calcium carbonate, (b) adding at least one acid having a pKa value of 0 or less at 20°C or having a pKa value from 0 to 2.5 at 20°C to the suspension of step a), and (c) treating the suspension of step (a) with carbon dioxide before, during or after step (b). According to another embodiment the surface-reacted calcium carbonate is obtained by a process comprising the steps of: (A) providing a natural or precipitated calcium carbonate, (B) providing at least one water-soluble acid, (C) providing gaseous $CO_2$, (D) contacting said natural or precipitated calcium carbonate of step (A) with the at least one acid of step (B) and with the $CO_2$ of step (C), characterised in that: (i) the at least one acid of step B) has a pKa of greater than 2.5 and less than or equal to 7 at 20°C, associated with the ionisation of its first available hydrogen, and a corresponding anion is formed on loss of this first available hydrogen capable of forming a water-soluble calcium salt, and (ii) following contacting the at least one acid with natural or precipitated calcium carbonate, at least one water-soluble salt, which in the case of a hydrogen-containing salt has a pKa of greater than 7 at 20°C, associated with the ionisation of the first available hydrogen, and the salt anion of which is capable of forming water-insoluble calcium salts, is additionally provided.

"Natural ground calcium carbonate" (GCC) preferably is selected from calcium carbonate containing minerals selected from the group comprising marble, chalk, limestone and mixtures thereof. Natural calcium carbonate may comprise further naturally occurring components such as alumino silicate etc.

In general, the grinding of natural ground calcium carbonate may be a dry or wet grinding step and may be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. The wet processed ground calcium carbonate containing mineral material thus obtained may be washed and dewatered by well-known processes, e.g. by flocculation, filtration or forced evaporation prior to drying. The subsequent step of drying (if necessary) may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and calcium hydroxide in an aqueous environment or by precipitation of calcium and carbonate ions, for example $CaCl_2$ and $Na_2CO_3$, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the precipitated calcium carbonate is precipitated calcium carbonate, preferably comprising aragonitic, vateritic or calcitic mineralogical crystal forms or mixtures thereof.

Precipitated calcium carbonate may be ground prior to the treatment with carbon dioxide and at least one $H_3O^+$ ion donor by the same means as used for grinding natural calcium carbonate as described above.

According to one embodiment of the present invention, the natural or precipitated calcium carbonate is in form of particles having a weight median particle size $d_{50}$ of 0.05 to 10.0 $\mu$m, preferably 0.2 to 5.0 $\mu$m, more preferably 0.4 to 3.0 $\mu$m, most preferably 0.6 to 1.2 $\mu$m, especially 0.7 $\mu$m. According to a further embodiment of the present invention, the natural or precipitated calcium carbonate is in form of particles having a top cut particle size $d_{98}$ of 0.15 to 55 $\mu$m, preferably 1 to 40 $\mu$m, more preferably 2 to 25 $\mu$m, most preferably 3 to 15 $\mu$m, especially 4 $\mu$m.

The natural and/or precipitated calcium carbonate may be used dry or suspended in water. Preferably, a corresponding slurry has a content of natural or precipitated calcium carbonate within the range of 1 wt.-% to 90 wt.-%, more preferably 3 wt.-% to 60 wt.-%, even more preferably 5 wt.-% to 40 wt.-%, and most preferably 10 wt.-% to 25 wt.-% based on the weight of the slurry.

The one or more $H_3O^+$ ion donor used for the preparation of surface reacted calcium carbonate may be any strong acid, medium-strong acid, or weak acid, or mixtures thereof, generating $H_3O^+$ ions under the preparation conditions. According to the present invention, the at least one $H_3O^+$ ion donor can also be an acidic salt, generating $H_3O^+$ ions under the preparation conditions.

According to one embodiment, the at least one $H_3O^+$ ion donor is a strong acid having a pKa of 0 or less at 20°C.

According to another embodiment, the at least one $H_3O^+$ ion donor is a medium-strong acid having a pKa value from 0 to 2.5 at 20°C. If the pKa at 20°C is 0 or less, the acid is preferably selected from sulphuric acid, hydrochloric acid, or mixtures thereof. If the pKa at 20°C is from 0 to 2.5, the $H_3O^+$ ion donor is preferably selected from $H_2SO_3$, $H_3PO_4$, oxalic acid, or mixtures thereof. The at least one $H_3O^+$ ion donor can also be an acidic salt, for example, $HSO_4^-$ or $H_2PO_4^-$, being at least partially neutralized by a corresponding cation such as $Li^+$, $Na^+$ or $K^+$, or $HPO_4^{2-}$, being at least partially neutralised by a corresponding cation such as $Li^+$, $Na^+$, $K^+$, $Mg^{2+}$ or $Ca^{2+}$. The at least one $H_3O^+$ ion donor can also be a mixture of one or more acids and one or more acidic salts.

According to still another embodiment, the at least one $H_3O^+$ ion donor is a weak acid having a pKa value of greater than 2.5 and less than or equal to 7, when measured at 20°C, associated with the ionisation of the first available hydrogen, and having a corresponding anion, which is capable of forming water-soluble calcium salts. Subsequently, at least one water-soluble salt, which in the case of a hydrogen-containing salt has a pKa of greater than 7, when measured at 20°C, associated with the ionisation of the first available hydrogen, and the salt anion of which is capable of forming water-insoluble calcium salts, is additionally provided. According to the preferred embodiment, the weak acid has a pKa value from greater than 2.5 to 5 at 20°C, and more preferably the weak acid is selected from the group consisting of acetic acid, formic acid, propanoic acid, and mixtures thereof. Exemplary cations of said water-soluble salt are selected from the group consisting of potassium, sodium, lithium and mixtures thereof. In a more preferred embodiment, said cation is sodium or potassium. Exemplary anions of said water-soluble salt are selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of drop wise addition, this addition preferably takes place within a time period of 10 minutes. It is more preferred to add said salt in one step.

According to one embodiment of the present invention, the at least one $H_3O^+$ ion donor is selected from the group consisting of hydrochloric acid, sulphuric acid, sulphurous acid, phosphoric acid, citric acid, oxalic acid, acetic acid, formic acid, and mixtures thereof. Preferably the at least one $H_3O^+$ ion donor is selected from the group consisting of hydrochloric acid, sulphuric acid, sulphurous acid, phosphoric acid, oxalic acid, $H_2PO_4^-$, being at least partially neutralised by a corresponding cation such as $Li^+$, $Na^+$ or $K^+$, $HPO_4^{2-}$, being at least partially neutralised by a corresponding cation such as $Li^+$, $Na^+$, $K^+$, $Mg^{2+}$, or $Ca^{2+}$ and mixtures thereof, more preferably the at least one acid is selected from the group consisting of hydrochloric acid, sulphuric acid, sulphurous acid, phosphoric acid, oxalic acid, or mixtures thereof, and most preferably, the at least one $H_3O^+$ ion donor is phosphoric acid.

The one or more $H_3O^+$ ion donor can be added to the suspension as a concentrated solution or a more diluted solution. Preferably, the molar ratio of the $H_3O^+$ ion donor to the natural or precipitated calcium carbonate is from 0.01 to 4, more preferably from 0.02 to 2, even more preferably 0.05 to 1 and most preferably 0.1 to 0.58.

As an alternative, it is also possible to add the $H_3O^+$ ion donor to the water before the natural or precipitated calcium carbonate is suspended.

In a preferred embodiment, the surface-reacted calcium carbonate is a reaction product of natural ground calcium carbonate with carbon dioxide and one or more $H_3O^+$ ion donors in an aqueous medium, wherein the carbon dioxide is formed in-situ by the $H_3O^+$ ion donors treatment and wherein the $H_3O^+$ ion donor is phosphoric acid. In a more preferred embodiment, the surface-reacted calcium carbonate is a reaction product of calcium carbonate containing minerals selected from the group comprising marble, chalk, limestone and mixtures thereof with carbon dioxide and one or more $H_3O^+$ ion donors in an aqueous medium, wherein the carbon dioxide is formed in-situ by the $H_3O^+$ ion donors treatment and wherein the $H_3O^+$ ion donor is phosphoric acid.

In a next step, the natural or precipitated calcium carbonate is treated with carbon dioxide. If a strong acid such as sulphuric acid or hydrochloric acid is used for the $H_3O^+$ ion donor treatment of the natural or precipitated calcium carbonate, the carbon dioxide is automatically formed. Alternatively or additionally, the carbon dioxide can be supplied from an external source.

$H_3O^+$ ion donor treatment and treatment with carbon dioxide can be carried out simultaneously which is the case when a strong or medium-strong acid is used. It is also possible to carry out $H_3O^+$ ion donor treatment first, e.g. with a medium strong acid having a pKa in the range of 0 to 2.5 at 20°C, wherein carbon dioxide is formed in-situ, and thus, the carbon dioxide treatment will automatically be carried out simultaneously with the $H_3O^+$ ion donor treatment, followed by the additional treatment with carbon dioxide supplied from an external source.

In a preferred embodiment, the $H_3O^+$ ion donor treatment step and/or the carbon dioxide treatment step are repeated at least once, more preferably several times. According to one embodiment, the at least one $H_3O^+$ ion donor is added over a time period of at least about 5 min, preferably at least about 10 min, typically from about 10 to about 20 min, more preferably about 30 min, even more preferably about 45 min, and sometimes about 1 h or more.

Subsequent to the $H_3O^+$ ion donor treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted natural or precipitated calcium carbonate as an aqueous suspension having a pH of greater than 6.0, preferably greater than 6.5, more preferably greater than 7.0, even more preferably greater than 7.5.

Further details about the preparation of the surface-reacted natural calcium carbonate are disclosed in WO0039222 A1, WO2004083316 A1, WO2005121257 A2, WO2009074492 A1, EP2264108 A1, EP2264109 A1 and US20040020410 A1, the content of these references herewith being included in the present application.

Similarly, surface-reacted precipitated calcium carbonate is obtained. As can be taken in detail from WO2009074492 A1, surface-reacted precipitated calcium carbonate is obtained by contacting precipitated calcium carbonate with $H_3O^+$ ions and with anions being solubilized in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by $H_3O^+$ ions, where said $H_3O^+$ ions are provided solely in the form of a counterion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in-situ.

Said $H_3O^+$ ions may be provided by the addition of an acid or an acid salt of said anion, or the addition of an acid or an acid salt which simultaneously serves to provide all or part of said excess solubilized calcium ions.

In a further preferred embodiment of the preparation of the surface-reacted natural or precipitated calcium carbonate, the natural or precipitated calcium carbonate is reacted with the acid and/or the carbon dioxide in the presence of at least one compound selected from the group consisting of silicate, silica, aluminium hydroxide, earth alkali aluminate such as sodium or potassium aluminate, magnesium oxide, or mixtures thereof. Preferably, the at least one silicate is selected from an aluminium silicate, a calcium silicate, or an earth alkali metal silicate. These components can be added to an aqueous suspension comprising the natural or precipitated calcium carbonate before adding the acid and/or carbon dioxide.

Alternatively, the silicate and/or silica and/or aluminium hydroxide and/or earth alkali aluminate and/or magnesium oxide component(s) can be added to the aqueous suspension of natural or precipitated calcium carbonate while the reaction of natural or precipitated calcium carbonate with an acid and carbon dioxide has already started. Further details about the preparation of the surface-reacted natural or precipitated calcium carbonate in the presence of at least one silicate and/or silica and/or aluminium hydroxide and/or earth alkali aluminate component(s) are disclosed in WO2004083316 A1, the content of this reference herewith being included in the present application.

The surface-reacted calcium carbonate can be kept in suspension, optionally further stabilised by a dispersant. Conventional dispersants known to the skilled person can be used. A preferred dispersant is comprised of polyacrylic acids and/or carboxymethylcelluloses.

Alternatively, the aqueous suspension described above can be dried, thereby obtaining the solid (i.e. dry or containing as little water that it is not in a fluid form) surface-reacted natural or precipitated calcium carbonate in the form of granules or a powder.

The surface reacted calcium carbonate may have different particle shapes, such as e.g. the shape of roses, golf balls and/or brains.

In a preferred embodiment, the surface-reacted calcium carbonate has a BET specific surface area of from 20 $m^2/g$ to 450 $m^2/g$, preferably from 20 $m^2/g$ to 250 $m^2/g$, more preferably from 30 $m^2/g$ to 160 $m^2/g$, most preferably from 40 $m^2/g$ to 150 $m^2/g$, still more preferably from 40 $m^2/g$ to 140 $m^2/g$ measured using the nitrogen and BET method according to ISO 9277:2010.

It is furthermore preferred that the surface-reacted calcium carbonate particles have a volume median particle diameter $d_{50}$ (or $d_{50}$ (vol)) of from 1 $\mu$m to 50 $\mu$m, preferably from 1 to 45 $\mu$m, more preferably from 2 to 30 $\mu$m.

It may furthermore be preferred that the surface-reacted calcium carbonate particles have a volume particle diameter $d_{98}$ (or $d_{98}$ (vol)) of from 2 to 150 $\mu$m, preferably from 4 to 100 $\mu$m, more preferably 6 to 80 $\mu$m, even more preferably from 8 to 60 $\mu$m, and most preferably from 10 to 30 $\mu$m.

The value $d_x$ represents the diameter relative to which x % of the particles have diameters less than $d_x$. This means that the $d_{98}$ value is the particle size at which 98 % of all particles are smaller. The $d_{98}$ value is also designated as "top cut". The $d_x$ values may be given in volume or weight percent. The $d_{50}$(wt) value is thus the weight median particle size, i.e. 50 wt.-% of all grains are smaller than this size, and the $d_{50}$ (vol) value is the volume median particle size, i.e. 50 vol.% of all grains are smaller than this particle size.

The "particle size" of surface-reacted calcium carbonate herein is described as volume-based particle size distribution, whereas the "particle size" of the granules herein is described as weight-based particle size distribution. Furthermore, the "particle size" of surface-reacted calcium carbonate in the meaning of the present invention refers to the primary particle size.

Volume median particle diameter $d_{50}$ was evaluated using a Malvern Mastersizer 2000 or 3000 Laser Diffraction System. The $d_{50}$ or $d_{98}$ value, measured using a Malvern Mastersizer 2000 or 3000 Laser Diffraction System, indicates a diameter value such that 50 % or 98 % by volume, respectively, of the particles have a diameter of less than this value. The raw data obtained by the measurement are analysed using the Mie theory, with a particle refractive index of 1.57 and an absorption index of 0.005.

Throughout the present invention, the weight-based particle size distribution, i.e. the weight-based particle size distribution of the granules, is determined by mechanical sieving. The measurement is made with a Retsch sieving shaker AS 200. The method and the instrument are known to the skilled person and are commonly used to determine grain size of fillers and pigments. The processes and instruments are known to the skilled person and are commonly used to determine grain size of fillers and pigments.

Preferably, the surface-reacted calcium carbonate has an intra-particle intruded specific pore volume within the range from 0.15 to 1.35 $cm^3/g$, preferably from 0.30 to 1.30 $cm^3/g$, more preferably from 0.30 to 1.25 $cm^3/g$, and most preferably from 0.30 to 0.90 $cm^3/g$ calculated from a mercury intrusion porosimetry measurement.

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 $\mu$m (~ nm). The equilibration time used at each pressure step is 20 seconds. The sample material is sealed in a 5 $cm^3$ chamber powder penetrometer for analysis. The data are corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 $\mu$m down to about 1 - 4 $\mu$m showing the coarse packing of the sample between any agglomerate structures contributing strongly. Below these diameters lies the fine interparticle packing of the particles themselves. If they also have intraparticle pores, then this region appears bi modal, and by taking the specific pore volume intruded by mercury into pores finer than the modal turning point, i.e. finer than the bi-modal point of inflection, we thus define the specific intraparticle pore volume. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

The intra-particle pore size of the surface-reacted calcium carbonate preferably is in a range of from 0.004 to 1.6 $\mu$m, more preferably in a range of between 0.005 to 1.3 $\mu$m, especially preferably from 0.006 to 1.15 $\mu$m and most preferably of 0.007 to 1.0 $\mu$m, e.g. 0.004 to 0.16 $\mu$m determined by mercury porosimetry measurement.

According to an exemplary embodiment, the surface-reacted calcium carbonate has

i) a BET specific surface area of from 20 m$^2$/g to 450 m$^2$/g, preferably from 20 m$^2$/g to 250 m$^2$/g, more preferably from 30 m$^2$/g to 160 m$^2$/g, most preferably from 40 m$^2$/g to 150 m$^2$/g, still more preferably from 40 m$^2$/g to 140 m$^2$/g measured using the nitrogen and BET method according to ISO 9277:2010, and/or

ii) a volume median particle diameter $d_{50}$ of from 1 $\mu$m to 50 $\mu$m, preferably from 1 to 45 $\mu$m, more preferably from 2 to 30 $\mu$m, and/or

iii) an intra-particle intruded specific pore volume within the range from 0.15 to 1.35 cm$^3$/g, preferably from 0.30 to 1.30 cm$^3$/g, more preferably from 0.30 to 1.25 cm$^3$/g, and most preferably from 0.30 to 0.90 cm3/g calculated from a mercury intrusion porosimetry measurement.

Preferably, the surface-reacted calcium carbonate has

i) a BET specific surface area of from 20 m$^2$/g to 450 m$^2$/g, preferably from 20 m$^2$/g to 250 m$^2$/g, more preferably from 30 m$^2$/g to 160 m2/g, most preferably from 40 m$^2$/g to 150 m$^2$/g, still more preferably from 40 m$^2$/g to 140 m$^2$/g measured using the nitrogen and BET method according to ISO 9277:2010, or

ii) a volume median particle diameter $d_{50}$ of from 1 to 50 $\mu$m, preferably from 1 to 45 $\mu$m, more preferably from 2 to 30 $\mu$m, or

iii) an intra-particle intruded specific pore volume within the range from 0.15 to 1.35 cm$^3$/g, preferably from 0.30 to 1.30 cm$^3$/g, more preferably from 0.30 to 1.25 cm$^3$/g, and most preferably from 0.30 to 0.90 cm$^3$/g calculated from a mercury intrusion porosimetry measurement.

Alternatively, the surface-reacted calcium carbonate has

i) a BET specific surface area of from 20 m$^2$/g to 450 m$^2$/g, preferably from 20 m$^2$/g to 250 m$^2$/g, more preferably from 30 m$^2$/g to 160 m$^2$/g, most preferably from 40 m$^2$/g to 150 m$^2$/g, still more preferably from 40 m$^2$/g to 140 m$^2$/g measured using the nitrogen and BET method according to ISO 9277:2010, and

ii) a volume median particle diameter $d_{50}$ of from 1 $\mu$m to 50 $\mu$m, preferably from 1 to 45 $\mu$m, more preferably from 2 to 30 $\mu$m, and

iii) an intra-particle intruded specific pore volume within the range from 0.15 to 1.35 cm$^3$/g, preferably from 0.30 to 1.30 cm$^3$/g, more preferably from 0.30 to 1.25 cm$^3$/g, and most preferably from 0.30 to 0.90 cm$^3$/g calculated from a mercury intrusion porosimetry measurement.

Surface-reacted calcium carbonate comprises an water-insoluble, at least partially crystalline calcium salt of an anion of the at least one acid, which is formed on the surface of the natural ground calcium carbonate or precipitated calcium carbonate. According to one embodiment, the water-insoluble, at least partially crystalline salt of an anion of the at least one acid covers the surface of the natural ground calcium carbonate or precipitated calcium carbonate at least partially, preferably completely. Depending on the employed at least one acid, the anion may be sulphate, sulphite, phosphate, citrate, oxalate, acetate, formate and/or chloride.

It is further required that the granules comprise one or more binder(s).

In one embodiment of the present invention, the one or more binder(s) comprise(s), preferably consist(s) of, one binder. Alternatively, the one or more binder(s) comprise(s), preferably consist(s) of, two or more binders. For example, the one or more binder(s) comprise(s), preferably consist(s) of, two or three binders.

Preferably, the one or more binder(s) comprise(s), preferably consist(s) of, one binder.

The one or more binder(s) is/are selected from the group comprising synthetic polymers such as methylcellulose, ethylcellulose, sodium carboxymethylcellulose, hydroxypropyl methylcellulose (HPMC), hydroxypropylcellulose (HPC), ethylhydroxyethylcellulose (EHEC), polyvinyl pyrrolidone (PVP), polyethylene glycol (PEG), polyvinyl alcohols, polymethacrylates; and natural binders such as plant gums, e.g. acacia, tragacanth, sandarac, ghatti, karaya, locust bean, carnauba wax, and guar; proteins such as gelatin, casein, collagen; animal exudates such as shellac; beeswax, alginic acid, and mixtures thereof.

In one embodiment, polyvinylpyrrolidone (PVP) is used as binder, preferably as the sole binder.

In this respect, it is to note that, if required, the binder may also have disintegrating properties under certain conditions. Such binders, which are known in the art, e.g. physically modified starch such as starch 1500® of Colorcon or pregelatinized starch such as Lycatab PGS of Roequette, have binding properties under granulation conditions, but are able to disintegrate the granules by, e.g. moisture sorption and swelling, if required.

The granules comprise the one or more binder(s) in an amount of from 0.25 to 35 wt.-%, preferably of from 0.5 to 15 wt.-%, more preferably of from 0.5 to 10 wt.-%, even more preferably of from 1.0 to 10 wt.-%, most preferably of from 1.5 to 10 wt.-%, based on the total dry weight of the granules

Granules are obtained by a granulation process. Granulation, i.e. the process in which the primary powder particles are made to adhere to form larger, multiparticle entities is a process of collecting particles together by creating bonds between them e.g. by one or more binder(s).

One of the most important types of granulation is wet granulation, wherein granules are formed by the addition of a granulation liquid onto a powder bed which is under the influence of an impeller. The agitation resulting in the system along with the wetting of the components within the formulation results in the agglomeration of the primary powder particles to produce wet granules. The granulation liquid contains a solvent which must be volatile so that it can be removed by drying, and be non-toxic. Water mixed into the powders can form bonds between powder particles that are strong enough to lock them together. However, once the water dries, the powders may fall apart. Therefore, water may not be strong enough to create and hold a bond. In such instances, the granulation liquid includes a binder.

Regarding surface-reacted calcium carbonate, granules are generally known. For example, in EP2264108 A1 (WO2010146530 A1), it is mentioned that the surface-reacted calcium carbonate obtained from the process described therein may be in the form of a cake, granules or a powder, and also in several documents describing different uses of surface-reacted calcium carbonate, such as in water purification, as a controlled release carrier, in fast disintegrating dosage forms, or gastroretentive drug formulation and delivery systems (EP1975310 B1, EP1982759 B1, EP1974807 B1, EP1974806 B1, EP2589430 A1, WO2010037753 A1, EP2719373 A1, or EP2719376 A1), granules are generally mentioned.

Furthermore, WO2015181306 A1 refers to a method for the production of granules comprising surface-reacted calcium carbonate, where it was found that, if the pores of the porous structure of the surface-reacted calcium carbonate particles are first saturated with the granulation liquid, whereas the binder is added afterwards, not only the stability of the resulting granules may be increased, but also the amount of binder may be decreased.

Thus, surface-reacted calcium carbonate together with the one or more binder(s) can be granulated using various methods which are well known in the art.

Thus, it is appreciated that the granules of the present invention are obtained by granulation, i.e. the granules are obtained under agitation in an agitation device. The granulation is finished as soon as the desired granule sizes, or granule size distribution, respectively, have been achieved, whereupon movement of the granules by air may be continued.

The granulation equipment may be selected from the conventionally used ones for granulation purposes. Thus, the agitation device may be selected from the group comprising Eirich mixers, fluidized bed dryers/granulators, plate granulators, table granulators, drum granulators, disc granulators, dish granulators, ploughshare mixer, vertical or horizontal mixers, high or low shear mixer, high speed blenders and rapid mixer granulators.

It might be noted that there may be differences as regards the granule sizes or granule size distributions to be achieved depending on the method used or the speed of mixing.

For example, the use of a fluidized bed mixer for granulation appears to provide a more uniform granule size distribution than the Lödige mixer, whereas the Lödige mixer gives a wider size distribution. Thus, multiple size ranges may be provided.

It is preferred that the granules are prepared in a fluidized bed mixer.

The resulting granules may have a wide size range, wherein different size fractions may be separated by conventional means such as sieving such as to obtain the granules with the required particle size distribution.

The granules of the present invention may comprise and/or may be mixed with at least one active ingredient and/or inactive precursor thereof.

That is to say, if the granules comprise at least one active ingredient and/or inactive precursor thereof, the surface-reacted calcium carbonate and the one or more binder(s) are preferably admixed with the at least one active ingredient and/or inactive precursor thereof before granulation such that the at least one active ingredient and/or inactive precursor thereof is distributed throughout the granules. Such granules are typically obtainable in a fluidized bed dryer/granulator, which are well known in the art. Alternatively, such granules are obtainable by any method suitable for preparing granules. It is appreciated that the obtained granules are preferably further subjected to a tabletting step via wet granulation in order to obtain the excipient of the present invention.

However, if the at least one active ingredient and/or inactive precursor thereof is a liquid, the granules are preferably mixed with the at least one active ingredient and/or inactive precursor thereof. In this case, the granules are thus preferably loaded with the at least one active ingredient and/or inactive precursor thereof.

If the granules are "mixed with" the at least one active ingredient and/or inactive precursor thereof, the surface-reacted calcium carbonate and the one or more binder(s) are granulated and the obtained granules are subjected to a treatment with the at least one active ingredient and/or inactive precursor thereof such that the at least one active ingredient and/or inactive precursor thereof is substantially only present on the outer surface of the granules. The granules which are subjected to a treatment with the at least one active ingredient and/or inactive precursor thereof are typically subjected to a compression process such as a direct compression process, well known in the art, in order to obtain the excipient of the present invention. The term "active ingredient" in the meaning of the present invention refers to a substance having a specific effect in an organism and causing a specific reaction in humans, animals, microorganisms and/or plants.

It is preferred that the at least one active ingredient and/or inactive precursor thereof is/are provided in liquid form.

The term "liquid" in the meaning of the present invention refers to a non-gaseous fluid composition, comprising or consisting of the at least one active ingredient and/or inactive precursor thereof, which is readily flowable at the pressure conditions and temperature of use, i.e. the pressure and temperature at which the granules are mixed with the at least one active ingredient and/or inactive precursor thereof.

Thus, it is appreciated that the at least one active ingredient and/or inactive precursor thereof can be liquid in a temperature range from 5 to 200°C, preferably from 10 to 120°C and most preferably from 10 to 100°C. For example, the at least one active ingredient and/or inactive precursor thereof can be liquid in a temperature range from 5 to 200°C, preferably from 10 to 120°C and most preferably from 10 to 100°C at ambient pressure conditions, i.e. at atmospheric pressure. Alternatively, the at least one active ingredient and/or inactive precursor thereof can be liquid in a temperature range from 5 to 200°C, preferably from 10 to 120°C and most preferably from 10 to 100°C at reduced pressure conditions, e.g. a pressure of from 100 to 700 mbar.

In one embodiment, the at least one active ingredient and/or inactive precursor thereof is/are liquid at ambient temperature and pressure conditions, e.g., at room temperature, such as from about 5 to 35°C, preferably from 10 to 30°C and most preferably from 15 to 25°C, and at atmospheric pressure.

Alternatively, the at least one active ingredient and/or inactive precursor thereof is/are molten at the temperature of use, e.g. from about 35 to 200°C, preferably from 45 to 120°C and most preferably from 55 to 100°C, and at ambient pressure conditions, i.e. at atmospheric pressure, or at reduced pressure conditions, e.g. a pressure of from 100 to 700 mbar.

Alternatively, the at least one active ingredient and/or inactive precursor thereof is/are dissolved in a solvent. That is to say, the at least one active ingredient and/or inactive precursor thereof and the solvent form a system in which no discrete solid particles are observed in the solvent and thus form a "solution".

In one embodiment of the present invention, the solvent is selected from the group comprising water, methanol, ethanol, n-butanol, isopropanol, n-propanol, acetone, dimethylsulphoxide, dimethylformamide, tetrahydrofurane, vegetable oils and the derivatives thereof, animal oils and the derivatives thereof, molten fats and waxes, and mixtures thereof. Preferably, the solvent is water, ethanol and/or acetone. More preferably, the solvent is water.

For example, the at least one active ingredient and/or inactive precursor thereof may be a chiral compound. Thus, the at least one active ingredient and/or inactive precursor thereof encompass the (R)-enantiomer, (S)-enantiomer and mixtures thereof, e.g. the racemic mixture.

Additionally, or alternatively, the at least one active ingredient and/or inactive precursor thereof may be an isomeric compound. Thus, the at least one active ingredient and/or inactive precursor thereof encompass the (Z)-isomer, (E)-isomer and mixtures thereof. For example, if it is stated that the active ingredient is cinnamaldehyde, the cinnamaldehyde may be present as (Z)-cinnamaldehyde and/or (E)-cinnamaldehyde.

For example, the at least one active ingredient and/or inactive precursor thereof is selected from the group comprising fragrances, flavours, herbal extracts and oils, fruit extracts and oils, nutrients, trace minerals, repellents, food, cosmetics, flame retardants, enzymes, macromolecules, pesticides, fertilizers, preserving agents, antioxidants, reactive chemicals, pharmaceutical and/or nutraceutical and/or veterinary active agents or pharmaceutical and/or nutraceutical and/or veterinary inactive precursors of synthetic origin, semi-synthetic origin, natural origin thereof, and mixtures thereof.

Fragrances are preferably alcohols, aldehydes and/or ketones having a molecular weight of at least about 100 g/mol and which are useful in imparting an odour, fragrance, essence, or scent either alone or in combination with other fragrances. For example, the fragrance can be selected from the group comprising 2,4-dimethyl-3-cyclohexene-1-methanol (floralol), 2,4-dimethyl cyclohexane methanol (dihydro floralol), 5,6-dimethyl-1-methylethenylbicyclo[2.2.1]hept-5-ene-2-methanol (arbozol), α,α,-4-trimethyl-3-cyclohexen-1-methanol (a-terpineol), 2,4,6-trimethyl-3-cyclohexene-1-methanol (isocyclo geraniol), 4-(1-methylethyl)cyclohexane methanol (mayol), α-3,3-trimethyl-2-norborane methanol, 1,1-dimethyl-1-(4-methylcyclohex-3-enyl)methanol, 2-phenylethanol, 2-cyclohexyl ethanol, 2-(o-methylphenyl)-ethanol, 2-(m-methylphenyl)ethanol, 2-(p-methylphenyl)ethanol, 6,6-dimethylbicyclo-[3.1.1]hept-2-ene-2-ethanol (nopol), 2-(4-methylphenoxy)-ethanol, 3,3-dimethyl-Δ2 -β-norbornane ethanol (patchomint), 2-methyl-2-cyclohexylethanol, 1-(4-isopropylcyclohexyl)-ethanol, 1-phenylethanol, 1,1-dimethyl-2-phenylethanol, 1,1-dimethyl-2-(4-methyl-phenyl)ethanol, 1-phenylpropanol, 3-phenylpropanol, 2-phenylpropanol (Hydrotropic Alcohol), 2-(cyclododecyl)propan-1-ol (Hydroxy-ambran), 2,2-dimethyl-3-(3-methylphenyl)-propan-1-ol (Majantol), 2-methyl-3-phenylpropanol, 3-phenyl-2-propen-1-ol (cinnamyl alcohol), 2-methyl-3-phenyl-2-propen-1-ol (methylcinnamyl alcohol), α-n-pentyl-3-phenyl-2-propen-1-ol (a-amyl-cinnamyl alcohol), ethyl-3-hydroxy-3-phenyl propionate, 2-(4-methylphenyl)-2-propanol, 3-(4-methylcyclohex-3-ene)butanol, 2-methyl-4-(2,2,3-trimethyl-3-cyclopenten-1-yl)butanol, 2-ethyl-4-(2,2,3-trimethyl-cyclopent-3-enyl)-2-buten-1-ol, 3-methyl-2-buten-1-ol (prenol), 2-methyl-4-(2,2,3-trimethyl-3-cyclopenten-1-yl)-2-buten-1-ol, ethyl 3-hydroxybutyrate, 4-phenyl-3-buten-2-ol, 2-methyl-4-phenylbutan-2-ol, 4-(4-hydroxyphenyl)butan-2-one, 4-(4-hydroxy-3-methoxyphenyl)-butan-2-one, 3-methyl-pentanol, 3-methyl-3-penten-1-ol, 1-(2-propenyl)cyclopentan-1-ol (plinol), 2-methyl-4-phenylpentanol (Pamplefleur), 3-methyl-5-phenylpentanol (Phenoxanol), 2-methyl-5-phenylpentanol, 2-methyl-5-(2,3-dimethyltricyclo[2.2.1.0.sup.(2,6) ]hept-3-yl)-2-penten-1-ol (santalol), 4-methyl-1-phenyl-2-pentanol, 5-(2,2,3-trimethyl-3-cyclopentenyl)-3-methylpentan-2-ol (sandalore), (1-methyl-bicyclo[2.1.1]hepten-2-yl)-2-methylpent-1-en-3-ol, 3-methyl-1-phenylpentan-3-ol, 1,2-dimethyl-3-(1-methylethenyl)cyclopentan-1-ol, 2-isopropyl-5-methyl-2-hexenol, cis-3-hexen-1-ol, trans-2-hexen-1-ol, 2-isoproenyl-4-methyl-4-hexen-1-ol (Lavandulol), 2-ethyl-2-prenyl-3-hexenol, 1-hydroxymethyl-4-isopropenyl-1-cyclohexene (Dihydrocuminyl alcohol), 1-methyl-4-isopropenylcyclohex-6-en-2-ol (carvenol), 6-methyl-3-isopropenylcyclohexan-1-ol (dihydrocarveol), 1-methyl-4-iso-propenylcyclohexan-3-ol, 4-isopropyl-1-methylcyclohexan-3-ol, 4-tert-butylcyclo-hexanol, 2-tert-butylcyclohexanol, 2-tert-butyl-4-methylcyclohexanol (rootanol), 4-isopropyl-cyclohexanol, 4-methyl-1-(1-methylethyl)-3-cyclohexen-1-ol, 2-(5,6,6-trimethyl-2-norbornyl)cyclohexanol, isobornylcyclohexanol, 3,3,5-trimethylcyclohexanol, 1-methyl-4-isopropylcyclohexan-3-ol, 1-methyl-4-isopropylcyclohexan-8-ol (dihydroterpineol), 1,2-dimethyl-3-(1-methylethyl)cyclohexan-1-ol, heptanol, 2,4-dimethylheptan-1-ol, 6-heptyl-5-hepten-2-ol (isolinalool), 2,4-dimethyl-2,6-heptandienol, 6,6-dimethyl-2-oxymethyl-bicyclo[3.1.1]hept-2-ene (myrtenol), 4-methyl-2,4-heptadien-1-ol, 3,4,5,6,6-pentamethyl-2-heptanol, 3,6-dimethyl-3-vinyl-5-hepten-2-ol, 6,6-dimethyl-3-hydroxy-2-methylenebicyclo[3.1.1]heptane, 1,7,7-trimethylbicyclo[2.2.1]heptan-2-ol, 2,6-dimethylheptan-2-ol (dimetol), 2,6,6-trimethylbicyclo[1.3.3]heptan-2-ol, octanol, 2-octenol, 2-methyloctan-2-ol, 2-methyl-6-methylene-7-octen-2-ol (myrcenol), 7-methyloctan-1-ol, 3,7-dimethyl-6-octenol, 3,7-dimethyl-7-octenol, 3,7-dimethyl-6-octen-1-ol (citronellol), 3,7-dimethyl-2,6-octadien-1-ol (geraniol), 3,7-dimethyl-2,6-octadien-1-ol (nerol), 3,7-dimethyl-7-methoxyoctan-2-ol (osyrol), 3,7-dimethyl-1,6-octadien-3-ol (linalool), 3,7-dimethyloctan-1-ol (pelargol), 3,7-dimethyloctan-3-ol (tetrahydrolinalool), 2,4-octadien-1-ol, 3,7-dimethyl-6-octen-3-ol (dihydrolinalool), 2,6-dimethyl-7-octen-2-ol (dihydromyrcenol), 2,6-dimethyl-5,7-octadien-2-ol, 4,7-dimethyl-4-vinyl-6-octen-3-ol, 3-methyloctan-3-ol, 2,6-dimethyloctan-2-ol, 2,6-dimethyloctan-3-ol, 3,6-dimethyloctan-3-ol, 2,6-dimethyl-7-octen-2-ol, 2,6-dimethyl-3,5-octadien-2-ol (muguol), 3-methyl-1-octen-3-ol, 7-hydroxy-3,7-dimethyloctanal, 3-nonanol, 2,6-nonadien-1-ol, cis-6-nonen-1-ol, 6,8-dimethylnonan-2-ol, 3-(hydroxymethyl)-2-nonanone, 2-nonen-1-ol, 2,4-nonadien-1-ol, 3,7-dimethyl-1,6-nonadien-3-ol, decanol, 9-decenol, 2-benzyl-M-dioxa-5-ol, 2-decen-1-ol, 2,4-decadien-1-ol, 4-methyl-3-decen-5-ol, 3,7,9-trimethyl-1,6-decadien-3-ol (isobutyl linalool), undecanol, 2-undecen-1-ol, 10-undecen-1-ol, 2-dodecen-1-ol, 2,4-dodecadien-1-ol, 2,7,11-trimethyl-2,6,10-dodecatrien-1-ol (farnesol), 3,7,11-trimethyl-1,6,10,-dodecatrien-3-ol (nerolidol), 3,7,11,15-tetramethylhexadec-2-en-1-ol (phytol), 3,7,11,15-tetramethylhexadec-1-en-3-ol (iso phytol), benzyl alcohol, p-methoxy benzyl alcohol (anisyl alcohol), para-cymen-7-ol (cuminyl alcohol), 4-methyl benzyl alcohol, 3,4-methylenedioxy benzyl alcohol, methyl salicylate, benzyl salicylate, cis-3-hexenyl salicylate, n-pentyl salicylate, 2-phenylethyl salicylate, n-hexyl salicylate, 2-methyl-5-isopropylphenol, 4-ethyl-2-methoxyphenol, 4-allyl-2-methoxyphenol (eugenol), 2-methoxy-4-(1-propenyl)phenol (isoeugenol), 4-allyl-2,6-dimethoxy-phenol, 4-tert-butylphenol, 2-ethoxy-4-methylphenol, 2-methyl-4-vinylphenol, 2-isopropyl-5-methylphenol (thymol), pentyl-ortho-hydroxy benzoate, ethyl 2-hydroxybenzoate, methyl 2,4-dihydroxy-3,6-dimethylbenzoate, 3-hydroxy-5-methoxy-1-methylbenzene, 2-tert-butyl-4-methyl-1-hydroxybenzene, 1-ethoxy-2-hydroxy-4-propenylbenzene, 4-hydroxytoluene, 4-hydroxy-3-methoxybenzaldehyde, 2-ethoxy-4-hydroxybenzaldehyde, decahydro-2-naphthol, 2,5,5-trimethyl-octahydro-2-naphthol, 1,3,3-trimethyl-2-norbornanol (fenchol), 3a,4,5,6,7,7a-hexahydro-2,4-dimethyl-4,7-methano-1H-inden-5-ol, 3a,4,5,6,7,7a-hexahydro-3,4-dimethyl-4,7-methano-1H-inden-5-ol, 2-methyl-2-vinyl-5-(1-hydroxy-1-methylethyl)tetrahydrofuran, β-caryophyllene alcohol, vanillin, ethyl vanillin, cinnamaldehyde, benzaldehyde, phenyl acetaldehyde, heptylaldehyde, octylaldehyde, decylaldehyde, undecylaldehyde, undecylenic aldehyde, dodecylaldehyde, tridecylaldehyde, methylnonyl aldehyde, didecylaldehyde, anisaldehyde, citronellal, citronellyloxyaldehyde, cyclamen aldehyde, α-hexyl cinnamaldehyde, hydroxycitronellal, α-methyl cinnamaldehyde, methylnonyl acetaldehyde, propylphenyl aldehyde, citral, perilla aldehyde, tolylaldehyde, tolylacetaldehyde, cuminaldehyde, LILIAL®, salicyl aldehyde, α-amylcinnamaldehyde and heliotropin and mixtures thereof.

Various essential oils, herbal extracts and/or fruit extracts may also be used, preferably those with various medicinal or dietary supplement properties. Essential oils, herbal extracts and/or fruit extracts are generally extracts or aromatic plants, plant parts, fruit or fruit parts that can be used medicinally or for flavouring. Suitable herbal extracts and/or fruit extracts can be used singly or in various mixtures. Commonly used essential oils, herbal extracts and/or fruit extracts include Echinacea, Goldenseal, Calendula, Rosemary, Thyme, Kava Kava, Aloe, Blood Root, Grapefruit Seed Extract, Black Cohosh, Ginseng, Guarana, Cranberry, Ginko Biloba, St. John's Wort, Evening Primrose Oil, Yohimbe Bark, Green Tea, Ma Huang, Maca, Bilberry, Lutein, Ginger, eugenol-containing oils and combinations thereof.

A variety of nutrients may be used including virtually any vitamin, mineral and/or phytochemical. For example, vitamin A, vitamin B1, vitamin B6, vitamin B12, vitamin B2, vitamin B6, vitamin D, vitamin E, i.e. tocopheroles, vitamin K, thiamine, riboflavin, biotin, folic acid, niacin, pantothenic acid, Q10, alpha lipoic acid, dihydrolipoic acid, curcumin, xanthophylls, beta cryptoxanthin, lycopene, lutein, zeaxanthin, astaxanthin, beta-carotene, carotenes, mixed carotenoids, polyphenols, flavonoids, sodium, potassium, calcium, magnesium, sulphur, chlorine, choline, and/or phytochemicals such as carotenoids, chlorophyll, chlorophyllin, fibre, flavanoids, anthocyanins, cyaniding, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof, may be used. Examples of nutrients that can be used as active ingredient(s) are set forth in U.S. Patent Application Publication Nos. 20030157213 A1, 20030206993 and 20030099741 A1.

In one embodiment, trace minerals can be used, e.g. manganese, zinc, copper, fluorine, molybdenum, iodine, cobalt, chromium, selenium, phosphorous, and combinations thereof

Enzymes can include but are not limited to coenzyme Q10, pepsin, phytase, trypsin, lipases, proteases, cellulases, lactase and combinations thereof.

Pesticides are preferably any known herbicide, insecticide, insect growth regulator, nematicide, termiticide, molluscicide, piscicide, avicide, rodenticide, predacide, bactericide, insect repellent, animal repellent, antimicrobial, fungicide, disinfectant (antimicrobial), and sanitizer known to the skilled person.

It is to be noted that the preserving agent may be any such compound known to the skilled person. For example, preserving agents may include, but are not limited to, phenoxyethanol, ethylhexylglycerin, parabens such as methyl paraben, ethyl paraben, propyl paraben, butyl paraben and mixtures thereof, benzalkonium chloride, chlorbutanol, benzyl alcohol, cetylpyridinium chloride, tartaric acid, lactic acid, malic acid, acetic acid, benzoic acid, sodium benzoate, sorbic acid, potassium sorbate and mixtures thereof.

Antioxidants are preferably selected from the group comprising butylhydroxyanisol (BHA), butylhydroxytoluol (BHT), gallate, carotinoid, polyphenols such as resveratrol, flavonoid and mixtures thereof, derivatives of polyphenols, tocopherol and salts thereof, betacarotin, ubichinon, tocotrienol, dihydroquercetin, antioxidants of natural origin and mixtures thereof. If the antioxidant is of natural origin, the antioxidant can be e.g. a conifer extract, pinus pinaster bark extract such as Pycnogenol® from Horphag, Switzerland, and/or emblica officinalis fruit extract such as Saberry® from Sabinsa corporation, Germany.

The pharmaceutically active agent or pharmaceutically inactive precursor thereof is preferably selected from the group comprising pharmaceutically active agent or pharmaceutically inactive precursor of synthetic origin, semi-synthetic origin, natural origin and combinations thereof.

Thus, a pharmaceutically active agent refers to pharmaceutically active agents which are of synthetic origin, semi-synthetic origin, natural origin and combinations thereof. Further, a pharmaceutically inactive precursor of the pharmaceutically active agent refers to pharmaceutically inactive precursors which are of synthetic origin, semi-synthetic origin, natural origin and combinations thereof and will be activated at a later stage to the respective pharmaceutically active agent.

The activation of such pharmaceutically inactive precursor is known to the skilled person and commonly in use, e.g. activation in the stomach and/or gastro-intestinal pathway- such as acidic activation or tryptic- or chimotryptic cleavage.

It lies within the understanding of the skilled person that the mentioned activation methods are of mere illustrative character and are not intended to be of limiting character.

It is to be noted that the pharmaceutically active agent or pharmaceutically inactive precursor thereof, may be any such compound known to the skilled person.

Pharmaceutically active agents thus include any compound that provides prophylactic and/or therapeutic properties when administered to humans and/or animals. Examples include, but are not limited to, pharmaceutical actives, therapeutic actives, veterinarian actives, nutraceuticals, and growth regulators.

The pharmaceutically active agent or pharmaceutically inactive precursor thereof can be an anti-inflammatory agent. Such agents may include, but are not limited to, non- steroidal anti-inflammatory agents or NSAIDs, such as propionic acid derivatives; acetic acid derivatives; fenamic acid derivatives; biphenylcarboxylic acid derivatives; and oxicams. All of these NSAIDs are fully described in U.S. Patent Number 4,985,459 to Sunshine et al.. Examples of useful NSAIDs include acetylsalicylic acid, ibuprofen, naproxen, benoxaprofen, flurbiprofen, fenoprofen, fenbufen, ketoprofen, indoprofen, pirprofen, carprofen, oxaprozin, pranoprofen, microprofen, tioxaprofen, suprofen, alminoprofen, tiaprofenic acid, fluprofen, bucloxic acid and mixtures thereof.

Also useful are the steroidal anti-inflammatory drugs such as hydrocortisone and the like, and COX-2 inhibitors such as meloxicam, celecoxib, rofecoxib, valdecoxib, etoricoxib or mixtures thereof. Mixtures of any of the above anti-inflammatories may be used.

Other materials that can be used as pharmaceutically active agent or pharmaceutically inactive precursor thereof include commonly known mouth and throat products. These products include, but are not limited to, upper respiratory agents such as phenylephrine, diphenhydramine, dextromethorphan, bromhexine and chiorpheniramine, gastrointestinal agents such as famotidine, loperamide and simethicone, anti-fungals such as miconazole nitrate, antibiotics and analgesics such as ketoprofen and fluribuprofen.

The pharmaceutically active agent or pharmaceutically inactive precursor thereof may be also selected from sodium pyrosulphite, butylhydroxytoluene, butylated hydroxyanisole.

The pharmaceutically active agent or pharmaceutically inactive precursor thereof may be also selected from ephedrine, magaldrate, pseudoephedrine, sildenafil, xylocaine, benzalconium chloride, caffeine, phenylephrine, amfepramone, orlistat, sibutramine, acetaminophen, aspirin, glitazones, metformin, chlorpromazine, dimenhydrinat, domperidone, meclozine, metoclopramide, odansetron, prednisolone, promethazine, acrivastine, cetirizine, cinnarizine, clemastine, cyclizine, desloratadine, dexchlorpheniramine, dimenhydrinate, ebastine, fexofenadine, ibuprofen, levolevoproricin, loratadine, meclozine, mizolastine, promethazine, miconazole, chlorhexidine diacetate, fluoride, decapeptide KSL, aluminium fluoride, aminochelated calcium, ammonium fluoride, ammonium fluorosilicate, ammonium monofluorphosphate, calcium fluoride, calcium gluconate, calcium glycerophosphate, calcium lactate, calcium monofluorphosphate, calciumcarbonate, carbamide, cetyl pyridinium chloride, chlorhexidine, chlorhexidine digluconate, chlorhexidine chloride, chlorhexidine diacetate, CPP caseine phospho peptide, hexetedine, octadecentyl ammonium fluoride, potassium fluorosilicate, potassium chloride, potassium monofluorphosphate, sodium bi carbonate, sodium carbonate, sodium fluoride, sodium fluorosilicate, sodium monofluorphosphate, sodium tri polyphosphate, stannous fluoride, stearyl trihydroxyethyl propylenediamine dihydrofluoride, strontium chloride, tetra potassium pyrophosphate, tetra sodium pyrophosphate, tripotassium orthophosphate, trisodium orthophosphate, alginic acid, aluminium hydroxide, sodium bicarbonate, sildenafil, tadalafil, vardenafil, yohimbine, cimetidine, nizatidine, ranitidine, acetylsalicylic acid, clopidogrel, acetylcysteine, bromhexine, codeine, dextromethorphan, diphenhydramine, noscapine, phenylpropanolamine, vitamin D, simvastatin, bisacodyl, lactitol, lactulose, magnesium oxide, sodium picosulphate, senna glycosides, benzocaine, lidocaine, tetracaine, almotriptan, eletriptan, naratriptan, rizatriptan, sumatriptan, zolmitriptan, calcium, chromium, copper, iodine, magnesium, manganese, molybdenium, phosphor, selenium, zinc, chloramine, hydrogen peroxide, metronidazole, triamcinolonacetonide, benzethonium chl., cetyl pyrid. chl., chlorhexidine, fluoride, lidocaine, amphotericin, miconazole, nystatin, fish oil, ginkgo biloba, ginseng, ginger, purple coneflower, saw palmetto, cetirizine, levocetirizine, loratadine, diclofenac, flurbiprofen, acrivastine pseudoephedrine, loratadine pseudoephedrine, glucosamine, hyaluronic acid, decapeptide KSL-W, decapeptide KSL, resveratrol, misoprostol, bupropion, ondansetron HCl, esomeprazole, lansoprazole, omeprazole, pantoprazole, rabeprazole, bacteria and the like, loperamide, simethicone, acetylsalicylic acid and others, sucralfate, clotrimazole, fluconazole, itraconazole, ketoconazole, terbinafine, allopurinol, probenecid, atorvastatin, fluvastatin, lovastatin, nicotinic acid, pravastatin, rosuvastatin, simvastatin, pilocarpine, naproxen, alendronate, etidronate, raloxifene, risedronate, benzodiazepines, disulphiram, naltrexone, buprenorphine, codeine, dextropropoxyphene, fentanyl, hydromorphone, ketobemidone, ketoprofen, methadone, morphine, naproxen, nicomorphine, oxycodone, pethidine, tramadol, amoxicillin, ampicillin, azithromycin, ciprofloxacin, clarithromycin, doxycyclin, erythromycin, fusidic acid, lymecycline, metronidazole, moxifloxacin, ofloxacin, oxytetracycline, phenoxymethylpenicillin, rifamycins, roxithromycin, sulphamethizole, tetracycline, trimethoprim, vancomycin, acarbose, glibenclamide, gliclazide, glimepiride, glipizide, insulin, repaglinide, tolbutamide, oseltamivir, aciclovir, famciclovir, penciclovir, valganciclovir, amlopidine, diltiazem, felodipine, nifedipine, verapamil, finasteride, minoxidil, cocaine, buphrenorpin, clonidine, methadone, naltrexone, calcium antagonists, clonidine, ergotamine, β-blockers, aceclofenac, celecoxib, dexiprofen, etodolac, indometacin, ketoprofen, ketorolac, lornoxicam, meloxicam, nabumetone, oiroxicam, parecoxib, phenylbutazone, piroxicam, tiaprofenic acid, tolfenamic acid, aripiprazole, chlorpromazine, chlorprothixene, clozapine, flupentixol, fluphenazine, haloperidol, lithium carbonate, lithium citrate, melperone, penfluridol, periciazine, perphenazine, pimozide, pipamperone, prochlorperazine, risperidone, thioridizin, fluconazole, itraconazole, ketoconazole, voriconazole, opium, benzodiazepines, hydroxine, meprobamate, phenothiazine, aluminiumaminoacetate, esomeprazole, famotidine, magnesium oxide, nizatide, omeprazole, pantoprazole, fluconazole, itraconazole, ketoconazole, metronidazole, amphetamine, atenolol, bisoprolol fumarate, metoprolol, metropolol, pindolol, propranolol, auranofin, and bendazac.

Further examples of useful pharmaceutically active agents or pharmaceutically inactive precursors thereof can include active ingredients selected from the therapeutical groups comprising: Analgesic, Anaesthetic, Antipyretic, Anti-allergic, Anti-arrhythmic, Appetite suppressant, Antifungal, Anti-inflammatory, Broncho dilator, Cardiovascular drugs, Coronary dilator, Cerebral dilator, Peripheral vasodilator, Anti-infective, Psychotropic, Anti-manic, Stimulant, Antihistamine, Laxative, Decongestant, Gastro-intestinal sedative, Sexual dysfunction agent, Disinfectants, Anti-diarrhoeal, Antianginal substance, Vasodilator, Anti-hypertensive agent, Vasoconstrictor, Migraine treating agent, Antibiotic, Tranquilizer, Antipsychotic, Anti-tumour drug, Anticoagulant, Antithrombotic agent, Hypnotic, Sedative, Anti-emetic, Anti-nauseant, Anticonvulsant, Neuromuscular agent, Hyper and hypoglycaemic, Thyroid and antithyroid, Diuretic, Antispasmodic, Uterine relaxant, Anti-obesity agent, Anorectic, Spasnolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretic, Anti-flatulent, Betablocker, Teeth Whitener, Enzyme, Co-enzyme, Protein, Energy booster, Fibre, Probiotics, Prebiotics, NSAID, Anti-tussives, Decongestants, Anti-histamines, Expectorants, Anti-diarrhoeals, Hydrogen antagonists, Proton pump inhibitors, General nonselective CNS depressants, General nonselective CNS stimulants, Selectively CNS function modifying drugs, Antiparkinsonism, Narcotic-analgetics, Analgetic-antipyretics, Psychopharmacological drugs, and Sexual dysfunction agents.

Examples of useful pharmaceutically active agents or pharmaceutically inactive precursors thereof may also include: Casein glyco-macro-peptide (CGMP), Triclosan, Cetyl pyridinium chloride, Domiphen bromide, Quaternary ammonium salts, zinc components, Sanguinarine, Fluorides, Alexidine, Octonidine, EDTA, Aspirin, Acetaminophen, Ibuprofen, Ketoprofen, Diflunisal, Fenoprofen calcium, Naproxen, Tolmetin sodium, Indomethacin, Benzonatate, Caramiphen edisylate, Menthol, Dextromethorphan hydrobromide, Theobromine hydrochloride, Chlophendianol Hydrochloride, Pseudoephedrine Hydrochloride, Phenylephrine, Phenylpropanolamine, Pseudoephedrine sulphate, Brompheniramine maleate, Chlorpheniramine- maleate, Carbinoxamine maleate, Clemastine fumarate, Dexchlorpheniramine maleate, Dephenhydramine hydrochloride, Diphenpyralide hydrochloride, Azatadine maleate, Diphenhydramine citrate, Doxylamine succinate, Promethazine hydrochloride, Pyrilamine maleate, Tripellenamine citrate, Triprolidine hydrochloride, Acrivastine, Loratadine, Brompheniramine, Dexbrompheniamine, Guaifenesin, Ipecac, potassium iodide, Terpin hydrate, Loperamide, Famotidine, Ranitidine, Omeprazole, Lansoprazole, Aliphatic alcohols, Barbiturates, caffeine, strychnine, Picrotoxin, Pentyenetetrazol, Phenyhydantoin, Phenobarbital, Primidone, Carbamazapine, Etoxsuximide, Methsuximide, Phensuximide, Trimethadione, Diazepam, Benzodiazepines, Phenacemide, Pheneturide, Acetazolamide, Sulthiame, bromide, Levodopa, Amantadine, Morphine, Heroin, Hydromorphone, Metopon, Oxymorphone, Levophanol, Codeine, Hydrocodone, Xycodone, Nalorphine, Naloxone, Naltrexone, Salicylates, Phenylbutazone, Indomethacin, Phenacetin, Chlorpromazine, Methotrimeprazine, Haloperidol, Clozapine, Reserpine, Imipramine, Tranylcypromine, Phenelzine, Lithium, Sildenafil citrate, Tadalafil, and Vardenafil CL. For example, eugenol can be used as anaesthetic.

Examples of useful pharmaceutically active agent or pharmaceutically inactive precursor thereof may include active ingredients selected from the groups of ace-inhibitors, antianginal drugs, anti- arrhythmias, anti-asthmatics, anticholesterolemics, analgesics, anaesthetics, anticonvulsants, anti-depressants, anti-diabetic agents, anti-diarrhoea preparations, antidotes, anti-histamines, anti-hypertensive drugs, anti-inflammatory agents, anti-lipid agents, anti- manics, anti-nauseants, anti-stroke agents, anti-thyroid preparations, anti-tumour drugs, anti- viral agents, acne drugs, alkaloids, amino acid preparations, anti-tussives, anti- uricemic drugs, anti-viral drugs, anabolic preparations, systemic and non-systemic anti- infective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocriptine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumour drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of useful pharmaceutically active agents or pharmaceutically inactive precursors thereof contemplated can also include antacids, H2-antagonists, and analgesics. For example, antacid dosages can be prepared using the ingredients calcium carbonate alone or in combination with magnesium hydroxide, and/or aluminium hydroxide. Moreover, antacids can be used in combination with H2-antagonists.

Analgesics include opiates and opiate derivatives, such as Oxycontin™, ibuprofen, aspirin, acetaminophen, and combinations thereof that may optionally include caffeine.

Other useful pharmaceutically active agents or pharmaceutically inactive precursors thereof can include anti-diarrhoeals such as Immodium™ AD, anti-histamines, anti-tussives, decongestants, vitamins, and breath fresheners. Also contemplated for use herein are anxiolytics such as Xanax™; anti-psychotics such as Clozaril™ and Haldol™; non-steroidal anti-inflammatories (NSAID's) such as ibuprofen, naproxen sodium, Voltaren™ and Lodine™, anti-histamines such as Claritin™, Hismanal™, Relafen™, and Tavist™; antiemetics such as Kytril™ and Cesamet™; bronchodilators such as Bentolin™, Proventil™; anti-depressants such as Prozac™, Zoloft™, and Paxil™; anti-migraines such as Imigra™, ACE-inhibitors such as Vasotec™, Capoten™ and Zestril™; anti- Alzheimer's agents, such as Nicergoline™; and CaH-antagonists such as Procardia™, Adalat™, and Calan™.

The popular H2-antagonists which are contemplated for use in the present invention include cimetidine, ranitidine hydrochloride, famotidine, nizatidine, ebrotidine, mifentidine, roxatidine, pisatidine and aceroxatidine.

Active antacid ingredients can include, but are not limited to, the following: aluminium hydroxide, dihydroxyaluminium aminoacetate, aminoacetic acid, aluminium phosphate, dihydroxyaluminium sodium carbonate, bicarbonate, bismuth aluminate, bismuth carbonate, bismuth subcarbonate, bismuth subgallate, bismuth subnitrate, bismuth subsilysilate, calcium phosphate, citrate ion (acid or salt), amino acetic acid, hydrate magnesium aluminate sulphate, magaldrate, magnesium aluminosilicate, magnesium carbonate, magnesium glycinate, magnesium hydroxide, magnesium oxide, magnesium trisilicate, milk solids, aluminium mono-ordibasic calcium phosphate, tricalcium phosphate, potassium bicarbonate, sodium tartrate, sodium bicarbonate, magnesium aluminosilicates, tartaric acids and salts.

In some embodiments, the pharmaceutically active agent or pharmaceutically inactive precursor thereof can be selected from analgesics/anaesthetics such as menthol, phenol, hexylresorcinol, benzocaine, dyclonine hydrochloride, benzyl alcohol, salicyl alcohol, and combinations thereof. In some embodiments, the pharmaceutically active agent or pharmaceutically inactive precursor thereof can be selected from demulcents such as slippery elm bark, pectin, gelatin, and combinations thereof. In some embodiments, the pharmaceutically active agent or pharmaceutically inactive precursor thereof can be selected from antiseptic ingredients such as cetylpyridinium chloride, domiphen bromide, dequalinium chloride, eugenol and combinations thereof.

In some embodiments, the pharmaceutically active agent or pharmaceutically inactive precursor thereof can be selected from antitussive ingredients such as chlophedianol hydrochloride, codeine, codeine phosphate, codeine sulphate, dextromethorphan, dextromethorphan hydrobromide, diphenhydramine citrate, and diphenhydramine hydrochloride, and combinations thereof.

In some embodiments, the pharmaceutically active agent or pharmaceutically inactive precursor thereof can be selected from throat soothing agents such as honey, propolis, aloe vera, glycerine, menthol and combinations thereof. In still other embodiments, the pharmaceutically active agent or pharmaceutically inactive precursor thereof can be selected from cough suppressants. Such cough suppressants can fall into two groups: those that alter the texture or production of phlegm such as mucolytics and expectorants; and those that suppress the coughing reflex such as codeine (narcotic cough suppressants), antihistamines, dextromethorphan and isoproterenol (non-narcotic cough suppressants).

In still other embodiments, the pharmaceutically active agent or pharmaceutically inactive precursor thereof can be an antitussive selected from the group comprising codeine, dextromethorphan, dextrorphan, diphenhydramine, hydrocodone, noscapine, oxycodone, pentoxyverine and combinations thereof. In some embodiments, the pharmaceutically active agent or pharmaceutically inactive precursor thereof can be selected from antihistamines such as acrivastine, azatadine, brompheniramine, chlo[phi]heniramine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof. In some embodiments, the pharmaceutically active agent or pharmaceutically inactive precursor thereof can be selected from non-sedating antihistamines such as astemizole, cetirizine, ebastine, fexofenadine, loratidine, terfenadine, and combinations thereof.

For example, the one or more active ingredient(s) is/are selected from fragrances, flavours, essential oils, insecticide, fungicide, pharmaceutically active agent, or pharmaceutically inactive precursor thereof, e.g. antiseptic and/or anaesthetic, and mixtures thereof.

If the granules comprise at least one active ingredient and/or inactive precursor thereof, the at least one active ingredient and/or inactive precursor thereof is/are preferably present in the granules in an amount from 0.5 to 80 wt.-%, preferably of from 10.0 to 70 wt.-% and most preferably of from 20 to 60 wt.-%, based on the total dry weight of the granules.

Additionally or alternatively, the granules comprise and/or are mixed with one or more lubricant(s) and/or one or more disintegrant(s).

For example, if the granules comprise one or more lubricant(s), the surface-reacted calcium carbonate and the one or more binder(s) are preferably admixed with the one or more lubricant(s) after granulation such that the one or more lubricant(s) is/are present on the surface of the granules.

Additionally or alternatively, if the granules comprise or more disintegrant(s), the surface-reacted calcium carbonate and the one or more binder(s) are preferably admixed with the one or more disintegrant(s) before granulation such that the one or more disintegrant(s) is/are present throughout the granules. Alternatively, the surface-reacted calcium carbonate and the one or more binder(s) are preferably admixed with the one or more disintegrant(s) after granulation such that the one or more disintegrant(s) is/are present on the surface of the granules.

Preferably, the granules comprise and/or are mixed with one lubricant and/or one disintegrant. For example, the granules comprise and/or are mixed with one lubricant or one disintegrant.

It is to be noted that the lubricant(s) and disintegrant(s) which may be used in the granules of the present invention generally are those well-known in the art of granulation.

In a preferred embodiment, the disintegrant may be selected from the group comprising sodium crosscarmellose, modified cellulose gums, insoluble cross-linked polyvinylpyrrolidones, starches, modified starches such as sodium starch glycolates, pregelatinized starch or sodium carboxymethyl starch, low-substituted hydroxypropyl cellulose, homopolymers of N-vinyl-2-pyrrolidone, alkyl-, hydroxyalkyl-, carboxyalkyl-cellulose esters, alginic acid, microcrystalline cellulose and its polymorphic forms, ion exchange resins, gums, chitin, chitosan, clays, gellan gum, crosslinked polacrillin copolymers, agar, dextrines, acrylic acid polymers, carboxymethylcellulose sodium/calcium, hydroxpropyl methyl cellulose phthalate, soy polysaccharide or mixtures thereof. The one or more disintegrant(s) can be also a superdisintegrant. The superdisintegrant(s) that may be used in the granules of the present invention generally are those well-known in the art of granulation. Exemplary superdisintegrants include but are not limited to sodium crosscarmellose, insoluble cross-linked polyvinylpyrrolidones , sodium starch glycolate, and mixtures thereto.

Lubricants may be selected from inner-phase lubricant(s) and/or outer-phase lubricant(s).

Such an inner-phase lubricant can be selected from the group comprising sorbitan esters of fatty acids and polyoxyethylated hydrogenated castor oil (e.g. the product sold under the trade name CREMOPHOR ®), block copolymers of ethylene oxide and propylene oxide (e.g. products sold under trade names PLURONIC ® and POLOXAMER), polyoxyethylene fatty alcohol ethers, polyoxyethylene sorbitan fatty acid esters, sorbitan esters of fatty acids and polyoxyethylene steraric acid esters, stearyl alcohol, glycerol dibehenate, sodium stearyl fumarate, glycerol distearate and combinations thereof. Preferably, the inner-phase lubricant is sodium stearyl fumarate.

The outer-phase lubricant can be selected from the group comprising lecithin, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllactylate, magnesium and/or calcium stearate, hydrogenated vegetable oils, stearic acid, sodium lauryl sulphate, magnesium lauryl sulphate, colloidal silica, talc and combinations thereof. Preferably, said outer-phase lubricant is magnesium and/or calcium stearate, more preferably magnesium stearate.

The one or more lubricant(s) is/are present in the granules in an amount of up to 5 wt.-%, based on the total dry weight of the granules. If the granules comprise and/or are mixed with one or more lubricant(s), the one or more lubricant(s) is/are present in the granules preferably from 0.2 to 5 wt.-%, more preferably from 0.2 to 4.5 wt.-%, even more preferably from 0.3 to 4.0 wt.-%, most preferably from 0.5 to 3.0 wt.-%, based on the total dry weight of the granules.

The one or more disintegrant(s) is/are present in the granules in an amount of up to 20 wt.-%, based on the total dry weight of the granules. If the granules comprise and/or are mixed with one or more disintegrant(s), the one or more disintegrant(s) is/are present in an amount preferably from 0.5 to 20 wt.-%, more preferably from 0.5 to 18 wt.-%, even more preferably from 1.0 to 16 wt.-%, most preferably from 1.5 to 12 wt.-%, based on the total dry weight of the granules. For example, if the one or more disintegrant(s) comprise(s) one or more superdisintegrant(s), preferably one superdisintegrant, the amount can be adjusted accordingly. In particular, if the one or more disintegrant(s) comprise one or more superdisintegrant(s), the one or more superdisintegrant(s), preferably one superdisintegrant, is/are present in an amount preferably of up to 8 wt.-%, preferably from 1.0 to 8 wt.-%, based on the total dry weight of the granules.

The granules comprising surface-reacted calcium carbonate and one or more binder(s) according to the present invention have turned out to be excellent as excipient in a pharmaceutical, nutraceutical, agricultural, veterinary, cosmetic, home, food, packaging or personal care product. It is preferred that the granules used for preparing the excipient are obtained in that the surface-reacted calcium carbonate and the one or more binder(s) are granulated and the obtained granules are subjected to a treatment with the at least one active ingredient and/or inactive precursor thereof such that the at least one active ingredient and/or inactive precursor thereof is substantially only present on the outer surface of the granules. It is appreciated that the granules, i.e. the granules subjected to a treatment with the at least one active ingredient and/or inactive precursor thereof, are then compressed in a compression process, preferably a direct compression process, to form the excipient. That is to say, the compression process, preferably the direct compression process, is carried out such that the granules are compressed into mini-tablets or tablets.

It is appreciated that the compression process can be carried out by any compression process known to the skilled person, for example with a tableting machine using single-punch or a rotary machine.

It is preferred that the compression process is a direct compression process. Preferably, a direct compression process using a force in the range from 1 to 40 kN, more preferably from 1.5 to 30 kN and most preferably from 1.5 to 25 kN.

It is appreciated that the compression process, preferably the direct compression process, results in mini-tablets or tablets suitable for a pharmaceutical, nutraceutical, agricultural, veterinary, cosmetic, home, food, packaging and personal care product.

Such mini-tablets or tablets are well known in the art and are of a particle size which is typically used for the products to be prepared.

For example, the mini-tablets or tablets have a weight median particle size $d_{50}$ of from 0.1 to 20.0 mm, preferably 0.2 to 15.0 mm and more preferably from 0.3 to 10.0 mm, as measured according to mechanical sieving.

Alternatively, if the granules are obtained in a fluidized bed granulator by mixing the surface-reacted calcium carbonate and the one or more binder(s) with at least one active ingredient and/or inactive precursor thereof before granulating, the obtained granules can be used as such as excipient in capsules or pliable packagings such as a sachet or flowpack, doypack, stickpack, and the like typically known. Thus, in one embodiment, the granules, i.e. granules obtained in a fluidized bed granulator by mixing the surface-reacted calcium carbonate and the one or more binder(s) with at least one active ingredient and/or inactive precursor thereof before granulating, are filled into capsules or pliable packagings such as a sachet or flowpack, doypack, stickpack, and the like. In one embodiment, the obtained granules, i.e. granules obtained in a fluidized bed granulator by mixing the surface-reacted calcium carbonate and the one or more binder(s) with at least one active ingredient and/or inactive precursor thereof before granulating, are further subjected to a tabletting step via wet granulation in order to obtain the excipient of the present invention.

The granules preferably provide an improved flowability and/or compactability .

The improvement in flowability and/or compactability is/are achieved if the ratio of hardness [N] to compression force [kN] (hardness/compression force) is at least 16, preferably at least 20. For example, the ratio of hardness [N] to compression force [kN] (hardness/compression force) is from 20 to 50, preferably from 20 to 40, and most preferably from 20 to 30.

Brief description of the Figures

Fig. 1 shows how to calculate the angle of repose ß.
Fig. 2 shows a comparison of the tablet hardness [N] of the mixtures as a function of the main compression force [kN].
Fig. 3 shows a comparison of the friability of the mixtures as a function tablet hardness [N].
Fig. 4 shows a comparison of the disintegration time of the mixtures as a function tablet hardness [N].
Fig. 5 shows a comparison of the tablet hardness [N] of the mixtures as a function of the main compression force [kN].
Fig. 6 shows a comparison of the friability of the sample 7 as a function tablet hardness [N].
Fig. 7 shows a comparison of the disintegration time of sample 7 as a function tablet hardness [N].

**Examples**

**1. Measurement methods**

In the following, measurement methods implemented in the examples are described.

**Particle size distribution**

Volume determined median particle size $d_{50}$(vol) and the volume determined top cut particle size $d_{98}$(vol) was evaluated using a Malvem Mastersizer 3000 Laser Diffraction System (Malvern Instruments Plc., Great Britain). The $d_{50}$(vol) or $d_{98}$(vol) value indicates a diameter value such that 50 % or 98 % by volume, respectively, of the particles have a diameter of less than this value. The raw data obtained by the measurement was analyzed using the Mie theory, with a particle refractive index of 1.57 and an absorption index of 0.005. The methods and instruments are known to the skilled person and are commonly used to determine particle size distributions of fillers and pigments. The sample was measured in dry condition without any prior treatment.

The weight determined median particle size $d_{50}$(wt) was measured by the sedimentation method, which is an analysis of sedimentation behaviour in a gravimetric field. The measurement was made with a Sedigraph™ 5120 of Micromeritics Instrument Corporation, USA. The method and the instrument are known to the skilled person and are commonly used to determine particle size distributions of fillers and pigments. The measurement was carried out in an aqueous solution of 0.1 wt.-% $Na_4P_2O_7$. The samples were dispersed using a high speed stirrer and supersonicated.

A vibrating sieve tower was used to analyse the particle size distribution of the granules. Aliquots of 120 g of granules were put on steel wire screens (Retsch, Germany) with mesh sizes of 90 pm, 180 pm, 250 pm, 355 pm, 500 pm, 710 pm, 5 and 1 mm. The sieving tower was shaken for 6 minutes with 10 seconds interval at a shaking displacement of 1 mm.

The processes and instruments are known to the skilled person and are commonly used to determine grain size of fillers and pigments.

**Specific surface area (SSA)**

The specific surface area was measured via the BET method according to ISO 9277:2010 using nitrogen, following conditioning of the sample by heating at 250°C for a period of 30 minutes. Prior to such measurements, the sample was filtered within a Büchner funnel, rinsed with deionised water and dried at 110°C in an oven for at least 12 hours.

**Intra-particle intruded specific pore volume (in cm³/g)**

The specific pore volume was measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 $\mu$m (~ nm). The equilibration time used at each pressure step was 20 seconds. The sample material was sealed in a 5 cm$^3$ chamber powder penetrometer for analysis. The data were corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 $\mu$m down to about 1 - 4 $\mu$m showing the coarse packing of the sample between any agglomerate structures contributing strongly. Below these diameters lies the fine inter-particle packing of the particles themselves. If they also have intra-particle pores, then this region appears bi-modal, and by taking the specific pore volume intruded by mercury into pores finer than the modal turning point, i.e. finer than the bi-modal point of inflection, the specific intra-particle pore volume is defined. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the inter-particle pore region and the intra-particle pore region, if present. Knowing the intra-particle pore diameter range it is possible to subtract the remainder inter-particle and inter-agglomerate pore volume from the total pore volume to deliver the desired pore volume of the internal pores alone in terms of the pore volume per unit mass

(specific pore volume). The same principle of subtraction, of course, applies for isolating any of the other pore size regions of interest.

**Bulk density**

100 $\pm$ 0.5 g of the respective material were carefully filled through a powder funnel into the 250 mL measuring cylinder and the volume was read off to the nearest 1 mL. The loose bulk density was the calculated according the formula:

$$\text{Loose bulk density [g/mL]} = \text{bulk volume [mL]/weighed sample [g]}$$

and the result was recorded to the nearest 0.01 g/mL.

**Tapped density**

100 $\pm$ 0.5 g of the respective material were carefully filled through a powder funnel into the 250 mL measuring cylinder.

The graduated cylinder is connected to a support provided with a settling apparatus capable of producing taps. The cylinder is secured in this support and the volume after 1 250 taps is read. A subsequent second tapping step consisting of 1 250 taps is performed and the value of the volume is read. When this second tapped volume value does not differ in more than 2 mL from this first tapped volume value, this is the tapped volume. When this value differs in more than 2 mL, the tapping step of 1 250 taps is repeated until no differences of more than 2 mL in subsequent steps is observed.

**Hausner Ratio**

The Hausner ratio is a number that is correlated to the flowability of a powder material and is calculated as follows:

$$\text{Hausner Ratio} = \text{(Tapped density) / (Bulk density)}$$

**Compressibility Index**

The compressibility index is calculated as follows:

$$\text{Compressibility Index (\%)} = \text{(Tapped density – Bulk density) / Tapped density} *100$$

**Angle of repose**

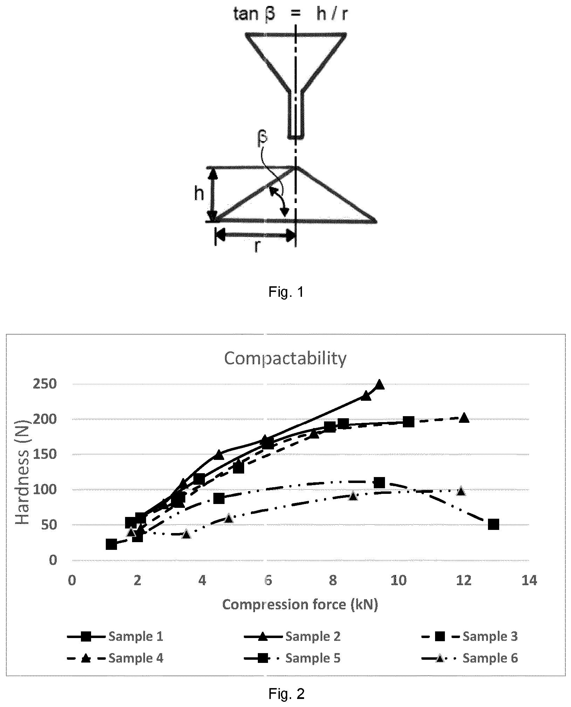

The angle of repose is measured in a flowability tester. The hopper equipped with the 10 mm nozzle is filled with approximately 150 mL of the respective material. After emptying the hopper, the granulate bevel is measured by means of a laser beam and the angle of repose is calculated. The angle of repose ß is the angle of the bevel flank opposite the horizontal line that is calculated as shown in Fig. 1:

**SEM**

The samples were prepared by diluting 50 to 150 $\mu$l slurry samples with 5 ml water. The amount of slurry sample depends on solids content, mean value of the particle size and particle size distribution. The diluted samples were filtrated by using a 0.8 $\mu$m membrane filter. A finer filter was used when the filtrate is turbid. A doubled-sided conductive adhesive tape was mounted on a SEM stub. This SEM stub was then slightly pressed in the still wet filter cake on the filter. The SEM stub was then sputtered with 8 nm Au. The investigation under the FESEM (Zeiss Sigma VP) was done at 5kV (Au). Subsequently, the prepared samples were examined by using a Sigma VP field emission scanning electron microscope (Carl Zeiss AG, Germany) and a secondary electron detector (SE2) at high vacuum (< $10^{-2}$ Pa).

**2. Materials used**

**Surface-reacted calcium carbonate**

SRCC

Surface-reacted calcium carbonate (SRCC) ($d_{50}$(vol) = 6.6 $\mu$m, $d_{98}$ = 13.7 $\mu$m, SSA = 59.9 $m^2$/g). The intra-particle intruded specific pore volume is 0.939 $cm^3$/g (for the pore diameter range of 0.004 to 0.51 $\mu$m).
SRCC was obtained by preparing 350 litres of an aqueous suspension of ground calcium carbonate in a mixing vessel by adjusting the solids content of a ground limestone calcium carbonate from Omya SAS, Orgon having a weight based median particle size $d_{50}$(wt) of 1.3 $\mu$m, as determined by sedimentation, such that a solids content of 10 wt.-%, based on the total weight of the aqueous suspension, is obtained.
Whilst mixing the slurry at a speed of 6.2 m/s, 11.2 kg phosphoric acid was added in form of an aqueous solution containing 30 wt.-% phosphoric acid to said suspension over a period of 20 minutes at a temperature of 70°C. After the addition of the acid, the slurry was stirred for additional 5 minutes, before removing it from the vessel and drying using a jet-dryer.

Other materials

Polyvinylpyrrolidone -K90 from BASF
Sodium crosscarmellose- Ac-di-sol, from JRS
Manesium stereate, Ligamed MF-2V, from Peter Greven
HPMC 4M, Methocel 4M, hydroxypropylmethylcellulose from Dupont

**3. Granulating FCC by fluid bed experiments**

**A. Using polyvinylpyrrolidone as binder**

Fluid-bed trials were performed on a GPC G2, Glatt, using a tangential spraying with the following settings:

- Air temp: 60°C
- Air Volume Flow.: 5-30 $m^3$/h
- Spray pump: 2-10 g/min
- Spray pressure 1.5 bar
- Batch size, surface reacted calcium carbonate: 500 g
- Polyvinylpyrrolidone (PVP) binder concentration: 4% w/w

Dry powder surface-reacted calcium carbonate (SRCC) were introduced in the fluid bed chamber in presence or absence of sodium crosscarmellose.
Table 1 shows proportions of polyvinylpyrrolidone (PVP) to surface-reacted calcium carbonate (SRCC) and sodium crosscarmellose (CCM) that have been used to prepare the excipients, i.e. granules:

Table 1: Composition of granules

| Sample No. | Surface-reacted calcium carbonate (SRCC) (%) | Sodium Crosscarmellose (CCM) (%*) | Polyvinylpyrrolidone (PVP) (%*) |
|---|---|---|---|
| 1 | 92.6 | 0 | 7.4 |
| 2 | 89.6 | 3 | 7.4 |
| *: % is given in weight-%, based on the total weight of the granules | | | |

The PSD of the granules are shown below in Table 2.

Table 2: PSD of different samples

| Sample No. | $d_{10}$ (μm) | $d_{50}$ (μm) | $d_{90}$ (μm) |
|---|---|---|---|
| 1 | 22 | 120 | 325 |
| 2 | 28 | 155.7 | 384.6 |

The density and compressibility values of the obtained co-processed excipients, i.e. granules, are listed in Table 3.

Table 3: Density and compressibility values

| Sample No. | Bulk density (g/ml) | Tapped density (g/ml) | Hausner ratio | Accord. EuPh | Angle of repose (°) |
|---|---|---|---|---|---|
| 1 | 0.41 | 0.49 | 1.20 | Fair | 40.1 |
| 2 | 0.41 | 0.47 | 1.15 | Good | 40.3 |

**Tabletting assays of fluid-bed granules (Samples 1 to 2)**

The granules set out in Tables 1 to 3 were further subjected to a tabletting step. The compositions of the tablets are set out in the following Table 4.

Table 4: Mixtures composition

| Sample No. | Fluid Bed Granules Sample 1 (%*) | Fluid Bed Granules Sample 2 (%*) | Sodium Crosscarmellose (%*) | Magnesium Stearate (%*) | Piroxicam (%*) | Caffeine (%*) |
|---|---|---|---|---|---|---|
| 1 | 95 | 0 | 3 | 2 | 0 | 0 |
| 2 | 0 | 98 | 0 | 2 | 0 | 0 |
| 3 | 65 | 0 | 3 | 2 | 0 | 30 |
| 4 | 0 | 68 | 0 | 2 | 0 | 30 |
| 5 | 75 | 0 | 3 | 2 | 20 | 0 |
| 6 | 0 | 78 | 0 | 2 | 20 | 0 |
| *: % is given in weight-%, based on the total weight of the tablets | | | | | | |

The obtained excipients, i.e. granules, (Samples no. 1 to 2) were further mixed with 2 wt.-% lubricant (Magnesium stearate, Ligamed MF-2-V, Cas# 557-04-0, Peter Greven) in a Turbula Mixer (Willy A. Bachofen, Turbula T10B) for 5 minutes. The mix was further used to prepare tablets in a Fette 1200i using EU1" tooling, a 10 mm fill cam, 8 standard convex round 10 mm punches and a tableting speed of 15000 tablets/hour. The fill depth was adjusted to obtain compression forces of 2 kN up to 20 kN and the tablet weight was fixed at 175 mg.

Alternatively, the obtained excipients were further mixed with a super disintegrant sodium crosscarmellose (CCM, Vivasol from JRS) and/or an active pharmaceutical ingredient, either caffeine anhydrous (BASF) or piroxicam (SelectChemie) in a Turbula Mixer (Willy A. Bachofen, Turbula T10B) for 10 minutes. These mixtures were further mixed with 2 wt.-% lubricant (Magnesium stearate, Ligamed MF-2-V, Cas# 557-04-0, Peter Greven) in a Turbula Mixer (Willy A.

Bachofen, Turbula T10B) for 5 minutes and used to prepare tablets as above.

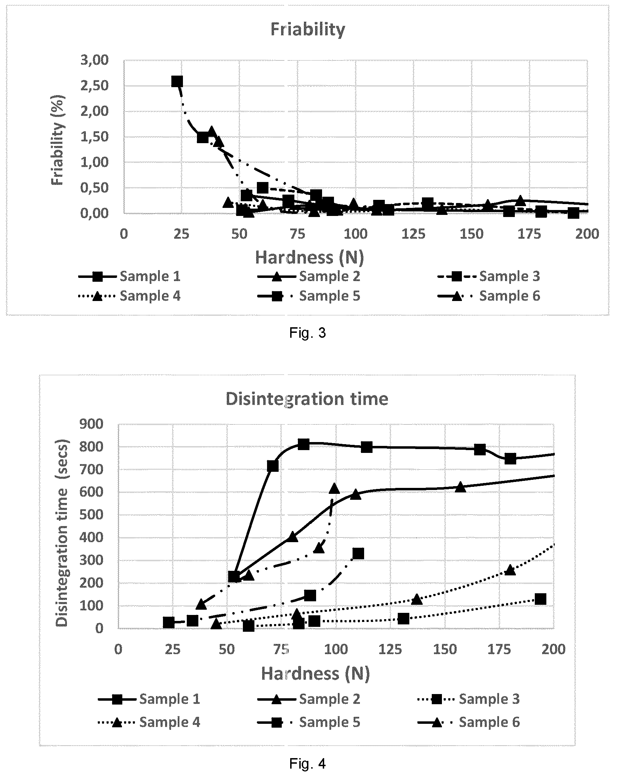

The tablet hardness [N] of the excipients as a function of the main compression force [kN] is shown in Fig. 2. The friability of the mixtures (%) as a function of the hardness [N] is shown in Fig. 3. Fig. 4 shows the disintegration time [sec] as a function of the tablet hardness [N] for the mixtures from table 4. The disintegration test was conducted with a DisiTest 50 Automatic Tablet Disintegration Tester of Pharmatron.

For the testing, a beaker was filled with 720 ml distilled water. The water was heated to 37.0°C, and then 6 Tablets were placed in a robust basket.

The apparatus automatically detects and records the disintegration time. In addition, the disintegration time was also monitored visually.

### B. Using hydroxypropylmethylcellulose as binder

Fluid-bed trials were performed on a GPC G2, Glatt, using a tangential spraying with the following settings:

- Air temp: 60°C
- Air Volume Flow.: 5-30 m3/h
- Spray pump: 2-10 g/min
- Spray pressure 1 bar
- Batch size, surface reacted calcium carbonate: 500 g
- Hydroxypropylmethylcellulose (HPMC) binder concentration: 0.5 % w/w

Dry powder surface-reacted calcium carbonate (SRCC) were introduced in the fluid bed chamber in presence or absence of sodium crosscarmellose.

Table 5 shows proportions of Hydroxypropylmethylcellulose (HPMC) to surface-reacted calcium carbonate (SRCC) and have been used to prepare the excipient:

Table 5: proportions of HPMC to SRCC

| Sample No. | Surface-reacted calcium carbonate (SRCC) (%*) | Hydroxypropylmethyl cellulose (HPMC) (%*) |
|---|---|---|
| 7 | 99 | 1 |
| *: % is given in weight-%, based on the total weight of the tablets | | |

The PSD of sample 7 is shown below in Table 6

Table 6: PSD of sample 7

| Sample No. | $d_{10}$ (μm) | $d_{50}$ (μm) | $d_{90}$ (μm) |
|---|---|---|---|
| 7 | 41 | 140 | 247 |

Table 7: Density and compressibility values

| Sample No. | Bulk density (g/ml) | Tapped density (g/ml) | Hausner ratio | Accord. EuPh | Mean flow (secs/100 g) |
|---|---|---|---|---|---|
| 7 | 0.370 | 0.46 | 1.24 | Fair | 10 |

The obtained excipients (sample 7) were further mixed with 10 % of caffeine anhydrous BASF) and 3 % of sodium crsscarmellose (CCM, Vivasol from JRS) ) in a Turbula Mixer (Willy A. Bachofen, Turbula T10B) for 10 minutes. Then, 2 wt.-% lubricant (Magnesium stearate, Ligamed MF-2-V, Cas# 557-04-0, Peter Greven) were added to the mixture and further mixed in a Turbula Mixer (Willy A. Bachofen, Turbula T10B) for 5 minutes. The mix was further used to prepare tablets in a Fette 1200i using EU1" tooling, a 10 mm fill cam, 8 standard convex round 10 mm punches and a tableting speed of 15000 tablets/hour. The fill depth was adjusted to obtain compression forces of 2 kN up to 20 kN and the tablet weight was fixed at 130 mg.

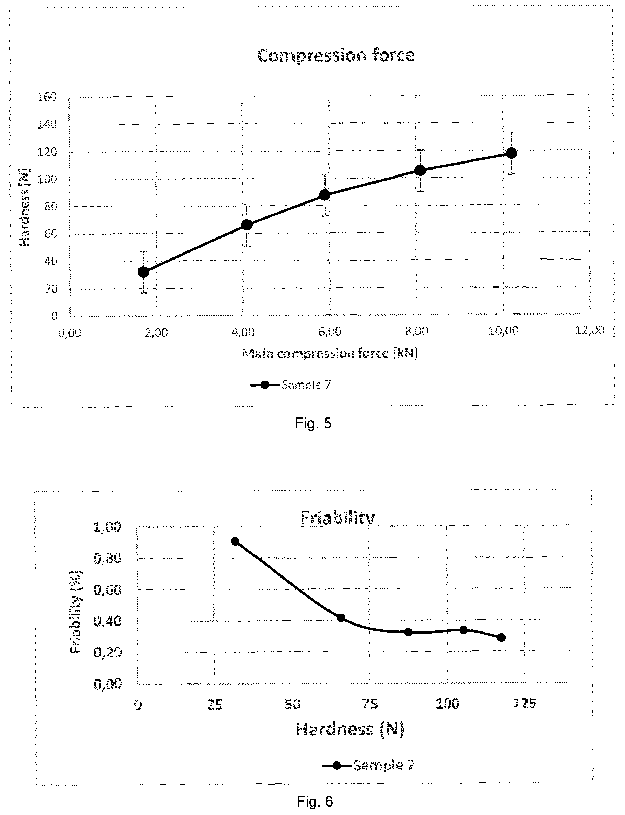

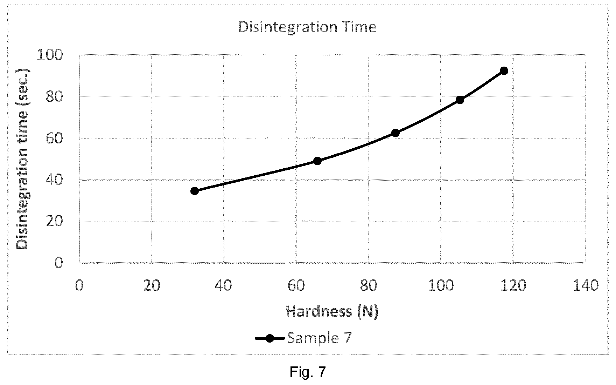

The results are shown in Fig. 5 to 7. Fig. 5 shows a comparison of the tablet hardness [N] of the mixtures as a function of the main compression force [kN]. Fig. 6 shows a comparison of the friability of the sample 7 as a function tablet hardness [N]. Fig. 7 shows a comparison of the disintegration time of sample 7 as a function tablet hardness [N].

**Claims**

1. Use of granules comprising surface-reacted calcium carbonate and one or more binder(s) as excipient in a pharmaceutical, nutraceutical, agricultural, veterinary, cosmetic, home, food, packaging or personal care product, wherein the granules have

   i) a weight particle size $d_{90}$ of 150 to 700 $\mu$m, as measured according to mechanical sieving,
   ii) a weight median particle size $d_{50}$ of 45 to 300 $\mu$m, as measured according to mechanical sieving,
   iii) a weight particle size $d_{10}$ of 18 to 100 $\mu$m, as measured according to mechanical sieving, and
   iv) a specific surface area of $\geq$ 15.0 m$^2$/g as measured by the BET nitrogen method,
   wherein the one or more binder(s) is/are synthetic polymers selected from methylcellulose, ethylcellulose, sodium carboxymethylcellulose, sodium crosscarmellose, hydroxypropyl methylcellulose (HPMC), hydroxypropylcellulose (HPC), ethylhydroxyethylcellulose (EHEC), polyvinyl pyrrolidone (PVP), polyethylene glycol (PEG), polyvinyl alcohols, polymethacrylates; and natural binders selected from plant gums, selected from acacia, tragacanth, sandarac, ghatti, karaya, locust bean, carnauba wax, and guar; proteins selected from gelatin, casein, collagen, animal exudates selected from shellac; beeswax, alginic acid, and mixtures thereof, and wherein the granules comprise the one or more binder(s) in an amount of from 0.25 to 35 wt.-%, based on the total dry weight of the granules.

2. The use according to claim 1, wherein the surface-reacted calcium carbonate is a reaction product of natural ground or precipitated calcium carbonate with carbon dioxide and one or more $H_3O^+$ ion donors in an aqueous medium, wherein the carbon dioxide is formed in-situ by the $H_3O^+$ ion donor treatment and/or is supplied from an external source.

3. The use according to claim 2, wherein the natural ground calcium carbonate is selected from calcium carbonate containing minerals selected from the group comprising marble, chalk, limestone and mixtures thereof; and that the precipitated calcium carbonate is selected from the group comprising precipitated calcium carbonates having aragonitic, vateritic or calcitic mineralogical crystal forms or mixtures thereof.

4. The use according to any one of the preceding claims, wherein the surface-reacted calcium carbonate has

   i) a BET specific surface area of from 20 m$^2$/g to 450 m$^2$/g, preferably from 20 m$^2$/g to 250 m$^2$/g, more preferably from 30 m$^2$/g to 160 m$^2$/g, most preferably from 40 m$^2$/g to 150 m$^2$/g, still more preferably from 40 m$^2$/g to 140 m$^2$/g measured using the nitrogen and BET method according to ISO 9277:2010, and/or
   ii) a volume median particle diameter $d_{50}$ of from 1 $\mu$m to 50 $\mu$m, preferably from 1 to 45 $\mu$m, more preferably from 2 to 30 $\mu$m, and/or
   iii) an intra-particle intruded specific pore volume within the range from 0.15 to 1.35 cm$^3$/g, preferably from 0.30 to 1.30 cm$^3$/g, more preferably from 0.30 to 1.25 cm$^3$/g, and most preferably from 0.30 to 0.90 cm$^3$/g calculated from a mercury intrusion porosimetry measurement.

5. The use according to any one of the preceding claims, wherein the granules comprise the one or more binder(s) in an amount of from 0.5 to 15 wt.-%, more preferably of from 0.5 to 10 wt.-%, even more preferably of from 1.0 to 10 wt.-%, most preferably of from 1.5 to 10 wt.-%, based on the total dry weight of the granules.

6. The use according to any one of the preceding claims, wherein the granules comprise and/or are mixed with at least one active ingredient and/or inactive precursor thereof, preferably selected from the group comprising fragrances, flavours, herbal extracts and oils, fruit extracts and oils, nutrients, trace minerals, repellents, food, cosmetics, flame retardants, enzymes, macromolecules, pesticides, fertilizers, preserving agents, antioxidants, reactive chemicals, pharmaceutical and/or nutraceutical and/or veterinary active agents or pharmaceutical and/or nutraceutical and/or veterinary inactive precursors of synthetic origin, semi-synthetic origin, natural origin thereof, and mixtures thereof, and/or one or more lubricant(s) and/or one or more disintegrant(s).

7. The use according to claim 6, wherein the granules comprise the at least one active ingredient and/or inactive precursor thereof in an amount from 0.5 to 80 wt.-%, preferably of from 10.0 to 70 wt.-% and most preferably of from 20 to 60 wt.-%, based on the total dry weight of the granules.

8. The use according to any one of the preceding claims, wherein the granules are obtained under agitation in an agitation device, preferably an agitation device selected from Eirich mixers, fluidized bed dryers/granulators, plate granulators, table granulators, drum granulators, disc granulators, dish granulators, ploughshare mixer, vertical or horizontal mixers, high or low shear mixer, high speed blenders and rapid mixer granulators.

9. The use according to any one of the preceding claims, wherein the granules have an intra-granular specific pore volume within the range from 0.15 to 2.75 cm$^3$/g, preferably from 0.30 to 2.50 cm$^3$/g, and most preferably from 0.40 to 2.00 cm$^3$/g, calculated from a mercury intrusion porosimetry measurement.

10. The use according to any one of the preceding claims, wherein the granules are compressed in a compression process into mini-tablets or tablets.

11. The use according to claim 10, wherein the granules are compressed in a direct compression process, preferably using a force in the range from 1 to 40 kN, more preferably from 1.5 to 30 kN and most preferably from 1.5 to 25 kN.

12. The use according to any one of claims 1 to 9, wherein the granules are filled into capsules or pliable packagings such as a sachet or flowpack, doypack, stickpack, and the like.

## Patentansprüche

1. Verwendung von Granulaten, umfassend oberflächenreagiertes Calciumcarbonat und ein oder mehrere Bindemittel als Hilfsstoff in einem pharmazeutischen, nutrazeutischen, landwirtschaftlichen, veterinärmedizinischen, kosmetischen, Haushalts-, Lebensmittel-, Verpackungs- oder Körperpflegeprodukt, wobei die Granulate

   i) eine Gewichts-Partikelgröße $d_{90}$ von 150 bis 700 $\mu$m, gemessen gemäß mechanischem Sieben,
   ii) eine gewichtsmittlere Partikelgröße $d_{50}$ von 45 bis 300 $\mu$m, gemessen durch mechanisches Sieben,
   iii) eine Gewichts-Partikelgröße $d_{10}$ von 18 bis 100 $\mu$m, gemessen nach mechanischer Siebung, und
   iv) eine spezifische Oberfläche von $\geq$ 15,0 m$^2$/g, gemessen nach der BET-Stickstoffmethode,

   aufweisen,
   wobei das eine oder die mehreren Bindemittel synthetische Polymere ist/sind, ausgewählt aus Methylcellulose, Ethylcellulose, Natriumcarboxymethylcellulose, Natriumcroscarmellose, Hydroxypropylmethylcellulose (HPMC), Hydroxypropylcellulose (HPC), Ethylhydroxyethylcellulose (EHEC), Polyvinylpyrrolidon (PVP), Polyethylenglykol (PEG), Polyvinylalkoholen, Polymethacrylaten; und natürlichen Bindemitteln ausgewählt aus Pflanzengummi ausgewählt aus Akazie, Tragant, Sandarak, Ghatti, Karaya, Johannisbrot, Carnaubawachs und Guar; Proteinen ausgewählt aus Gelatine, Kasein, Kollagen, tierischen Exsudaten, ausgewählt aus Schellack; Bienenwachs, Alginsäure und Mischungen davon, und wobei die Granulate das eine oder mehrere Bindemittel in einer Menge von 0,25 bis 35 Gew.-%, bezogen auf das Gesamttrockengewicht der Granulate, umfassen.

2. Verwendung gemäß Anspruch 1, wobei das oberflächen reagierte Calciumcarbonat ein Reaktionsprodukt aus natürlichem gemahlenem oder gefälltem Calciumcarbonat mit Kohlendioxid und einem oder mehreren $H_3O^+$-Ionendonatoren in einem wässrigen Medium ist, wobei das Kohlendioxid in situ durch die Behandlung mit dem $H_3O^+$-Ionendonator gebildet und/oder aus einer externen Quelle zugeführt wird.

3. Verwendung gemäß Anspruch 2, wobei das natürliche gemahlene Calciumcarbonat aus Calciumcarbonat enthaltenden Mineralien ausgewählt ist aus der Gruppe umfassend Marmor, Kreide, Kalkstein und Mischungen davon; und wobei das gefällte Calciumcarbonat aus der Gruppe ausgewählt ist, umfassend gefällte Calciumcarbonate mit aragonitischen, vateritischen oder calcitischen mineralogischen Kristallformen oder Mischungen davon.

4. Verwendung gemäß einem der vorstehenden Ansprüche, wobei das oberflächenreagierte Calciumcarbonat

   i) eine BET-spezifische Oberfläche von 20 m$^2$/g bis 450 m$^2$/g, vorzugsweise von 20 m$^2$/g bis 250 m$^2$/g, noch bevorzugter von 30 m$^2$/g bis 160 m$^2$/g, am meisten bevorzugt von 40 m$^2$/g bis 150 m$^2$/g, noch bevorzugter von 40 m$^2$/g bis 140 m$^2$/g, gemessen unter Verwendung von Stickstoff und der BET-Methode gemäß ISO 9277:2010, und/oder
   ii) einen volumenmittleren Partikeldurchmesser $d_{50}$ von 1 $\mu$m bis 50 $\mu$m, vorzugsweise von 1 bis 45 $\mu$m, noch bevorzugter von 2 bis 30 $\mu$m, und/oder
   iii) ein intrapartikuläres spezifisches Porenvolumen im Bereich von 0,15 bis 1,35 cm$^3$/g, vorzugsweise von 0,30 bis 1,30 cm$^3$/g, noch bevorzugter von 0,30 bis 1,25 cm$^3$/g und am meisten bevorzugt von 0,30 bis 0,90 cm$^3$/g, berechnet aus einer Quecksilber-Intrusionsporosimetriemessung,

aufweist.

**5.** Verwendung gemäß einem der vorstehenden Ansprüche, wobei die Granulate das eine oder mehrere Bindemittel in einer Menge von 0,5 bis 15 Gew.-%, vorzugsweise von 0,5 bis 10 Gew.-%, noch bevorzugter von 1,0 bis 10 Gew.-%, am meisten bevorzugt von 1,5 bis 10 Gew.-%, bezogen auf das Gesamttrockengewicht der Granulate, umfassen.

**6.** Verwendung gemäß einem der vorstehenden Ansprüche, wobei die Granulate mindestens einen Wirkstoff und/oder einen inaktiven Vorläufer davon umfassen und/oder damit gemischt sind, vorzugsweise ausgewählt aus der Gruppe umfassend Duftstoffe, Aromen, Kräuterextrakte und -öle, Fruchtextrakte und -öle, Nährstoffen, Spurenelemente, Repellentien, Lebensmitteln, Kosmetika, Flammschutzmittel, Enzyme, Makromoleküle, Pestizide, Düngemittel, Konservierungsmittel, Antioxidantien, reaktive Chemikalien, pharmazeutische und/oder nutrazeutische und/oder veterinärmedizinische Wirkstoffe oder pharmazeutische und/oder nutrazeutische und/oder veterinärmedizinische inaktive Vorläufer synthetischen, halbsynthetischen oder natürlichen Ursprungs und Mischungen davon und/oder einem oder mehreren Schmiermittel(n) und/oder einem oder mehreren Sprengmittel(n).

**7.** Verwendung gemäß Anspruch 6, wobei die Granulate mindestens einen Wirkstoff und/oder inaktiven Vorläufer davon in einer Menge von 0,5 bis 80 Gew.-%, vorzugsweise von 10,0 bis 70 Gew.-% und am meisten bevorzugt von 20 bis 60 Gew.-%, bezogen auf das Gesamttrockengewicht der Granulate, umfassen.

**8.** Verwendung gemäß einem der vorstehenden Ansprüche, wobei die Granulate unter Rühren in einer Rührvorrichtung erhalten werden, vorzugsweise einer Rührvorrichtung, die ausgewählt ist aus Eirich-Mischern, Fließbetttrocknern/-granulatoren, Plattengranulatoren, Tischgranulatoren, Trommelgranulatoren, Scheibengranulatoren, Schüsselgranulatoren, Pflugscharmischern, Vertikal- oder Horizontalmischern, Hoch- oder Niedrigscher-Mischern, Hochgeschwindigkeitsmischern und Schnellmischgranulatoren.

**9.** Verwendung gemäß einem der vorstehenden Ansprüche, wobei das Granulat ein intragranuläres spezifisches Porenvolumen im Bereich von 0,15 bis 2,75 cm$^3$/g, vorzugsweise von 0,30 bis 2,50 cm$^3$/g, und am meisten bevorzugt von 0,40 bis 2,00 cm$^3$/g, berechnet aus einer Quecksilber-Intrusionsporosimetriemessung, aufweist.

**10.** Verwendung gemäß einem der vorstehenden Ansprüche, wobei die Granulate in einem Kompressionsverfahren zu Minitabletten oder Tabletten gepresst werden.

**11.** Verwendung gemäß Anspruch 10, wobei die Granulate in einem Direktkompressionsverfahren komprimiert werden, vorzugsweise unter Verwendung einer Kraft im Bereich von 1 bis 40 kN, noch bevorzugter von 1,5 bis 30 kN und am meisten bevorzugt von 1,5 bis 25 kN.

**12.** Verwendung gemäß einem der Ansprüche 1 bis 9, wobei die Granulate in Kapseln oder biegsame Verpackungen wie beispielsweise einen Beutel oder ein Flowpack, Doypack, Stickpack und dergleichen abgefüllt werden.

**Revendications**

**1.** Utilisation de granulés comprenant du carbonate de calcium traité par réaction en surface et un ou plusieurs liant(s) en tant qu'excipient dans un produit pharmaceutique, nutraceutique, agricole, vétérinaire, cosmétique, domestique, alimentaire, de conditionnement ou de soins personnels, dans laquelle les granulés ont

i) un diamètre de particule en poids $d_{90}$ de 150 à 700 $\mu$m, mesuré par un tamisage mécanique,
ii) un diamètre de particule médian en poids $d_{50}$ de 45 à 300 $\mu$m, mesuré par un tamisage mécanique,
iii) un diamètre de particule en poids $d_{10}$ de 18 à 100 $\mu$m, mesuré par un tamisage mécanique, et
iv) une surface spécifique $\geq$ 15,0 m$^2$/g, mesurée par la méthode BET à l'azote,
dans laquelle le ou les liants(s) est/sont des polymères synthétiques choisi parmi la méthylcellulose, l'éthylcellulose, la carboxyméthylcellulose sodique, la crosscarmellose sodique, l'hydroxypropylméthylcellulose (HPMC), l'hydroxypropylcellulose (HPC), l'éthylhydroxyéthylcellulose (EHEC), la polyvinylpyrrolidone (PVP), le polyéthylène glycol (PEG), les alcools polyvinyliques, les polyméthacrylates; et des liants naturels choisis parmi les gommes végétales, choisies parmi l'acacia, l'adragante, la sandaraque, le ghatti, le karaya, la caroube, la cire de carnauba et le guar ; des protéines choisies parmi la gélatine, la caséine, le collagène, des exsudats d'animaux choisis parmi la gomme laque ; la cire d'abeille, l'acide alginique et des mélanges de ceux-ci, et
dans laquelle les granulés comprennent un ou plusieurs liant(s) en une quantité de 0,25 à 35 % en poids, en se basant sur le poids total des granulés.

2. Utilisation selon la revendication 1, dans laquelle le carbonate de calcium traité par réaction en surface est un produit de réaction entre du carbonate de calcium naturel broyé ou précipité, du dioxyde de carbone et un ou plusieurs donneurs d'ions $H_3O^+$ dans un milieu aqueux, dans laquelle le dioxyde de carbone est formé in situ par traitement avec un donneur d'ions $H_3O^+$ et/ou provient d'une source externe.

3. Utilisation selon la revendication 2, dans laquelle le carbonate de calcium naturel broyé est choisi parmi des minéraux contenant du carbonate de calcium choisis dans le groupe comprenant le marbre, la craie, le calcaire et des mélanges de ceux-ci ; et le carbonate de calcium précipité est choisi dans le groupe comprenant les carbonates de calcium précipités ayant des formes cristallines minéralogiques aragonitiques, vatéritiques ou calcitiques ou des mélanges de ceux-ci.

4. Utilisation selon l'une quelconque des revendications précédentes, dans laquelle le carbonate de calcium traité par réaction en surface a

i) une surface spécifique BET de 20 m²/g à 450 m²/g, de préférence de 20 m²/g à 250 m²/g, plus préférablement de 30 m²/g à 160 m²/g, de manière préférée entre toutes de 40 m²/g à 150 m²/g, encore plus préférablement de 40 m²/g à 140 m²/g mesurée en utilisant de l'azote et la méthode BET selon la norme ISO 9277:2010, et/ou
ii) un diamètre de particule médian en volume $d_{50}$ de 1 μm à 50 μm, de préférence de 1 à 45 μm, plus préférablement de 2 à 30 μm, et/ou
iii) un volume poreux spécifique intrusif intraparticulaire compris dans la plage allant de 0,15 à 1,35 cm³/g, de préférence de 0,30 à 1,30 cm³/g, plus préférablement de 0,30 à 1,25 cm³/g, et de manière préférée entre toutes de 0,30 à 0,90 cm³/g calculé à partir d'une mesure par porosimétrie d'intrusion au mercure.

5. Utilisation selon l'une quelconque des revendications précédentes, dans laquelle les granulés comprennent le ou les liant(s) en une quantité de 0,5 à 15 % en poids, plus préférablement de 0,5 à 10 % en poids, encore plus préférablement de 1,0 à 10 % en poids, de manière préférée entre toutes de 1,5 à 10 % en poids, en se basant sur le poids total des granulés.

6. Utilisation selon l'une quelconque des revendications précédentes, dans laquelle les granulés comprennent et/ou sont mélangés à au moins un ingrédient actif et/ou précurseur inactif de celui-ci, de préférence choisi dans le groupe comprenant les parfums, les arômes, les extraits et huiles de plantes, les extraits et huiles de fruits, les nutriments, les oligo-éléments, les répulsifs, les aliments, les cosmétiques, les retardateurs de flamme, les enzymes, les macromolécules, les pesticides, les engrais, les agents de conservation, les antioxydants, les produits chimiques réactifs, les agents actifs pharmaceutiques et/ou nutraceutiques et/ou vétérinaires ou les précurseurs inactifs pharmaceutiques et/ou nutraceutiques et/ou vétérinaires d'origine synthétique, d'origine semi-synthétique, d'origine naturelle et des mélanges de ceux-ci, et/ou un ou plusieurs lubrifiant(s) et/ou un ou plusieurs désintégrant(s).

7. Utilisation selon la revendication 6, dans laquelle les granulés comprennent l'au moins un ingrédient actif et/ou précurseur inactif de celui-ci en une quantité de 0,5 à 80 % en poids, de préférence de 10,0 à 70 % en poids et de manière préférée entre toutes de 20 à 60 % en poids, en se basant sur le poids total des granulés.

8. Utilisation selon l'une quelconque des revendications précédentes, dans laquelle les granulés sont obtenus sous agitation dans un dispositif d'agitation, de préférence un dispositif d'agitation choisi parmi les mélangeurs Eirich, les séchoirs/granulateurs à lit fluidisé, les granulateurs à plaques, les granulateurs de table, les granulateurs à tambour, les granulateurs à disque, les granulateurs à coupelle, les mélangeurs à socs de charrue, les mélangeurs verticaux ou horizontaux, les mélangeurs à cisaillement élevé ou faible, les mélangeurs à grande vitesse et les granulateurs à mélange rapide.

9. Utilisation selon l'une quelconque des revendications précédentes, dans laquelle les granulés ont un volume poreux spécifique intrusif intraparticulaire compris dans la plage allant de 0,15 à 2,75 cm³/g, de préférence de 0,30 à 2,50 cm³/g, et de manière préférée entre toutes de 0,40 à 2,00 cm³/g, calculé à partir d'une mesure par porosimétrie d'intrusion au mercure.

10. Utilisation selon l'une quelconque des revendications précédentes, dans laquelle les granulés sont comprimés dans un processus de compression en mini-comprimés ou en comprimés.

11. Utilisation selon la revendication 10, dans laquelle les granulés sont comprimés dans un processus de compression directe, de préférence en utilisant une force comprise dans la plage allant de 1 à 40 kN, plus préférablement de 1,5 et 30 kN et de manière préférée entre toutes de 1,5 à 25 kN.

12. Utilisation selon l'une quelconque des revendications 1 à 9, dans laquelle les granulés sont conditionnés dans des capsules ou des emballages pliables tels qu'un sachet, un flowpack, un doypack, un stickpack, et analogues.

Fig. 1

Fig. 2

Fig. 3

Fig. 4

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016150773 A1
- WO 2016096997 A1
- WO 2020058252 A1
- WO 2017093437 A1
- WO 2015181306 A1
- WO 0039222 A1
- WO 2004083316 A1
- WO 2005121257 A2
- WO 2009074492 A1
- EP 2264108 A1
- EP 2264109 A1
- US 20040020410 A1
- WO 2010146530 A1
- EP 1975310 B1
- EP 1982759 B1
- EP 1974807 B1
- EP 1974806 B1
- EP 2589430 A1
- WO 2010037753 A1
- EP 2719373 A1
- EP 2719376 A1
- US 20030157213 A1
- US 20030206993 A1
- US 20030099741 A1
- US 4985459 A

### Non-patent literature cited in the description

- **GANE, P.A.C.** ; **KETTLE, J.P.** ; **MATTHEWS, G.P.** ; **RIDGWAY, C.J.** Void Space Structure of Compressible Polymer Spheres and Consolidated Calcium Carbonate Paper-Coating Formulations. *Industrial and Engineering Chemistry Research*, 1996, vol. 35 (5), 1753-1764

- **GANE, P.A.C.** ; **KETTLE, J.P** ; **MATTHEWS, G.P.** ; **RIDGWAY, C.J.** Void Space Structure of Compressible Polymer Spheres and Consolidated Calcium Carbonate Paper-Coating Formulations. *Industrial and Engineering Chemistry Research*, 1996, vol. 35 (5), 1753-1764